# EUROPÄISCHE PATENTSCHRIFT

(11) **EP 0 733 052 B1**
(45) Veröffentlichungstag und Bekanntmachung des Hinweises auf die Patenterteilung: **27.09.2000**
(21) Anmeldenummer: 95902103.1
(22) Anmeldetag: 30.11.1994
(51) Int. Cl.: C07D 403/06, C07D 403/04, C07D 239/96, C07D 243/14, A61K 31/505

(54) **BICYCLEN-DERIVATE, IHRE HERSTELLUNG UND VERWENDUNG**
BICYCLENE DERIVATIVES, THEIR PRODUCTION AND USE
DERIVES DU BICYCLENE, PREPARATION ET UTILISATION

(30) Priorität: 07.12.1993 DE 4341665
(43) Veröffentlichungstag der Anmeldung: 25.09.1996
(73) Patentinhaber: BASF AKTIENGESELLSCHAFT, 67056 Ludwigshafen (DE)
(72) Erfinder: KLINGE, Dagmar, D-69120 Heidelberg (DE); UNGER, Liliane, D-67065 Ludwigshafen (DE); RASCHACK, Manfred, D-67256 Weisenheim (DE); WERNET, Wolfgang, D-67454 Hassloch (DE)
(86) Internationale Anmeldenummer: EP9403980
(87) Internationale Veröffentlichungsnummer: WO9515963

(56) Entgegenhaltungen:
- EP-A- 0 456 835
- CHEMICAL ABSTRACTS, vol. 84, no. 28, 1976, Columbus, Ohio, US; abstract no. 44132h, Seite 505 ; & JP,A,7 595 287 (HISAMITSU) 29. Juli 1975

## Beschreibung

Die vorliegende Erfindung betrifft neue Bicyclen-Derivate, deren Herstellung und Verwendung in der Therapie.

Bicyclische Verbindungen, speziell Chinoxalin-2,4-dione sind bereits bekannt. So sind in C.A. 84, 44132h (1976) solche Verbindungen bekannt, die entzündungshemmende und schmerzlindernde Wirkungen besitzen sollen. In der EP-A-456.835 sind Chinoxalin-2,4-dione beschrieben, die die Thrombocytenaggregation und die Aldose-Reduktase hemmen. Diese Verbindungen sollen sich daher zur Bekämpfung von Herz-Kreislauf-Erkrankungen und von Diabetes mellitus eignen.

Endothelin ist ein aus 21 Aminosäuren aufgebautes Peptid, das von vaskulärem Endothel synthetisiert und freigesetzt wird. Endothelin existiert in drei Isoformen, ET-1, ET-2 und ET-3. Im Folgenden bezeichnet "Endothelin" oder "ET" eine oder alle Isoformen von Endothelin. Endothelin ist ein potenter Vasokonstriktor und hat einen starken Effekt auf den Gefäßtonus. Es ist bekannt, daß diese Vasokonstriktion von der Bindung von Endothelin an seinen Rezeptor verursacht wird (Nature, 332, 411-415, 1988; FEBS Letters, 231, 440-444, 1988 und Biochem. Biophys. Res. Commun., 154, 868-875, 1988).

Erhöhte oder abnormale Freisetzung von Endothelin verursacht eine anhaltende Gefäßkontraktion in peripheren, renalen und zerebralen Blutgefäßen, die zu Krankheiten führen kann. Wie in der Literatur berichtet, wurden erhöhte Plasmaspiegel von Endothelin gefunden bei Patienten mit Hypertonie, akutem Myokardinfarkt, pulmonärer Hypertonie, Raynaud-Syndrom, Atherosklerose und in den Atemwegen von Asthmatikern (Japan J. Hypertension, 12, 79 (1989), J. Vascular Med. Biology 2, 207 (1990), J. Am. Med. Association 264, 2868 (1990)).

Demnach sollten Substanzen, die spezifisch die Bindung von Endothelin an den Rezeptor inhibieren, auch die obengenannten verschiedenen physiologischen Effekte von Endothelin antagonisieren und daher wertvolle Pharmaka darstellen.

Es wurde nun gefunden, daß bestimmte Bicyclen-Derivate gute Endothelin-antagonistische Aktivität besitzen.

Gegenstand der Erfindung ist die Verwendung von Bicyclen-Derivaten der Formel I 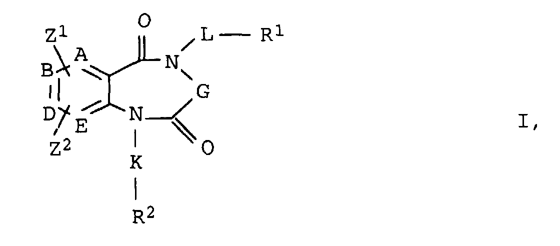 worin
2 der Reste A, B, D, E CH-Gruppen und die 2 anderen Reste CH-Gruppen oder Stickstoffatome bedeuten,
- Z¹: ein Wasserstoff- oder Halogenatom, eine C₁₋₆-Alkyl-gruppe, eine gegebenenfalls am aromatischen Rest durch C₁₋₆-Alkyl, C₁₋₆-Alkoxy, Halogen, CF₃, NO₂ oder CN substituierte Phenyl-, Phenyl-C₁₋₄-alkylen-, Naphthyl-, Naphthyl-C₁₋₆-alkylen-Gruppe, eine C₂₋₆-Alkenyl- oder C₂₋₆-Alkinylgruppe, eine C₃₋₇-Cycloalkylgruppe oder eine der Gruppen-NHR⁴, -NR⁴₂, -OR⁴, -SO₂NHR⁴, -SO₂NR⁴₂, -COR⁴ oder -CO₂R⁴ (mit R⁴ in der Bedeutung von C₁₋₄-Alkyl, Phenyl, Phenyl-C₁₋₄-alkylen, Naphthyl oder Naphthyl-C₁₋₄-alkylen) ist,
- Z²: eine der für Z¹ angegebenen Bedeutungen besitzt, jedoch kein Wasserstoffatom ist, oder
- Z¹: und Z² zusammen mit B und D auch einen der Reste 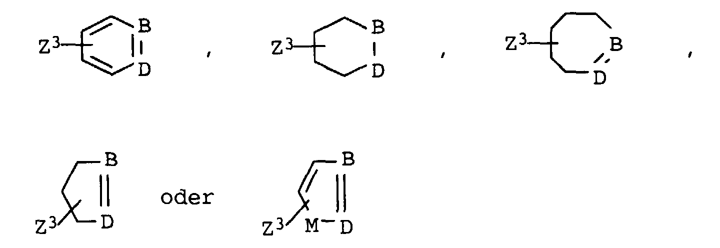 (worin Z³ eine der für Z¹ angegebenen Bedeutungen besitzt und M eine CH₂- oder NH-Gruppe ist) bedeuten,
- G: eine direkte Bindung oder die Gruppe CH-K¹ darstellt (mit K¹ in der Bedeutung von Wasserstoff, C₁₋₆-Alkyl, einer gegebenenfalls im Arylteil durch C₁₋₄-Alkyl, C₁₋₄-Alkoxy, Halogen, CF₃, NO₂ oder CN substituierten Phenyl-, Benzyl-, Naphthyl- oder Naphthylmethylen-Gruppe),
- K: eine Alkylen- oder Alkenylgruppe mit jeweils bis zu 6 C-Atomen oder die Gruppe 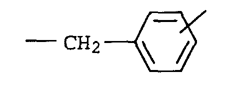 ist,
- L: eine Alkylen-, Alkenylen- oder Alkinylengruppe mit jeweils bis zu 6 C-Atomen oder eine der Gruppen 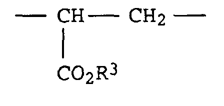 (mit R³ in der Bedeutung eines Wasserstoffatoms, einer C₁₋₄-Alkyl-, B 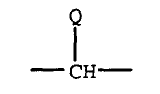 enzyl- oder Naphthylmethylengruppe), [mit Q in der Bedeutung von C₁-C₆-Alkyl, Aryl oder CH₂-R⁷, worin R⁷ Phenyl, 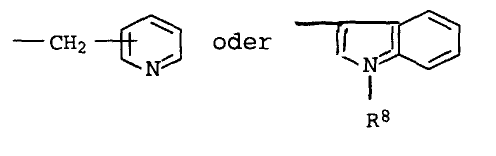 (R⁸= H, C₁₋₃-Alkyl, -CHO, -COO-C₁₋₃-Alkyl) bedeutet] oder 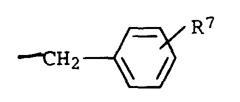
- R₁: eine -CO₂R⁹-Gruppe (mit R⁹ in der Bedeutung von Wasserstoff, C₁₋₄-Alkyl oder Benzyl), -CONR⁹₂, -OR⁹, -SR⁹, -SO₃R⁹, -PO₃R⁹₂ oder einen Tetrazolrest und
- R²: die Gruppe 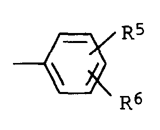 (mit R⁵ und R⁶ in der Bedeutung von Wasserstoff, C₁₋₄-Alkyl, -OR⁹ oder -SR⁹), 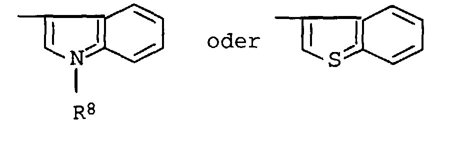
darstellen, sowie gegebenenfalls deren Salzen mit physiologisch verträglichen Säuren zur Herstellung von Arzneimitteln zur Bekämpfung von Krankheiten, die auf einem erhöhten Endothelinspiegel beruhen.

### Gegenstand der Erfindung sind weiter

Bicyclen-Derivate der Formel I 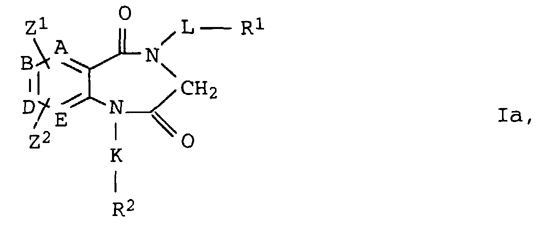 worin
2 der Reste A, B, D, E CH-Gruppen und die 2 anderen Reste CH-Gruppen oder Stickstoffatome bedeuten,
- Z¹: ein Wasserstoff- oder Halogenatom, eine C₁₋₆-Alkyl-gruppe, eine gegebenenfalls am aromatischen Rest durch C₁₋₆-Alkyl, C₁₋₆-Alkoxy, Halogen, CF₃, NO₂ oder CN substituierte Phenyl-, Phenyl-C₁₋₄alkylen-, Naphthyl-, Naphthyl-C₁₋₆-alkylen-Gruppe, eine C₂₋₆-Alkenyl- oder C₂₋₆-Alkinyl-gruppe, eine C₃₋₇-Cycloalkylgruppe oder eine der Gruppen-NHR⁴, -NR⁴₂, -OR⁴, -SO₂NHR⁴, -SO₂NR⁴₂, -COR⁴ oder -CO₂R⁴ (mit R⁴ in der Bedeutung von C₁₋₄-Alkyl, Phenyl, Phenyl-C₁₋₄-alkylen, Naphthyl oder Naphthyl-C₁₋₄-alkylen) ist,
- Z²: eine der für Z¹ angegebenen Bedeutungen besitzt, jedoch kein Wasserstoffatom ist, oder
- Z¹: und Z² zusammen mit B und D auch einen der Reste 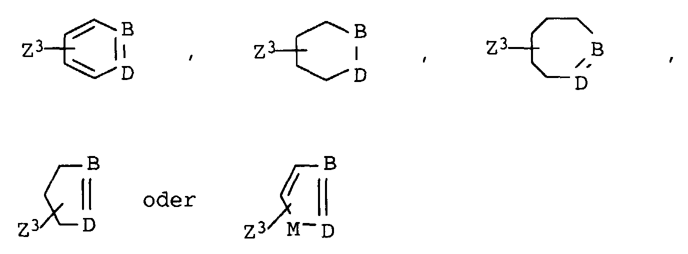 (worin Z³ eine der für Z¹ angegebenen Bedeutungen besitzt und M eine CH₂- oder NH-Gruppe ist) bedeuten,
- K: eine Alkylen- oder Alkenylgruppe mit jeweils bis zu 6 C-Atomen oder die Gruppe 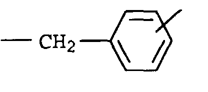 ist,
- L: eine Alkylen-, Alkenylen- oder Alkinylengruppe mit jeweils bis zu 6 C-Atomen oder eine der Gruppen 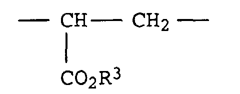 (mit R³ in der Bedeutung eines Wasserstoffatoms, einer C₁₋₄-Alkyl-, Benzyl- oder Naphthylmethylengruppe), 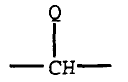 [mit Q in der Bedeutung von C₁-C₆-Alkyl, Aryl oder CH₂-R⁷, worin R⁷ Phenyl 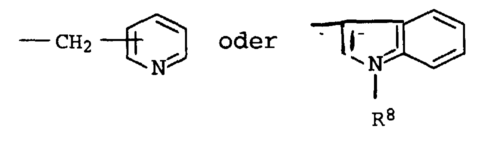 (R⁸= H, C₁₋₃-Alkyl, -CHO, -COO-C₁₋₃-Alkyl) bedeutet] oder 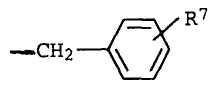
- R¹: eine -CO₂R⁹-Gruppe (mit R⁹ in der Bedeutung von Wasserstoff, C₁₋₄-Alkyl oder Benzyl), -CONR⁹₂, -OR⁹, -SR⁹, -SO₃R⁹, -PO₃R⁹₂ oder einen Tetrazolrest und
- R²: die Gruppe 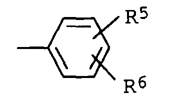 (mit R⁵ und R⁶ in der Bedeutung von Wasserstoff, C₁₋₄-Alkyl, -OR⁹ oder -SR⁹), 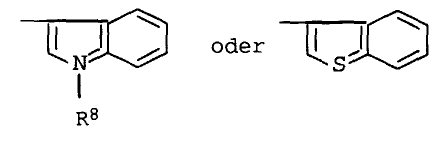
darstellen, sowie gegebenenfalls deren Salze mit physiologisch verträglichen Säuren
sowie

Bicyclen-Derivate der Formel I 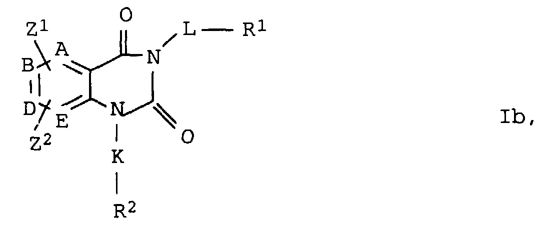 worin
2 der Reste A, B, D, E CH-Gruppen und die 2 anderen Reste CH-Gruppen oder Stickstoffatome bedeuten,
- Z¹: ein Wasserstoff- oder Halogenatom, eine C₁₋₆-Alkyl-gruppe, eine gegebenenfalls am aromatischen Rest durch C₁₋₆-Alkyl, C₁₋₆-Alkoxy, Halogen, CF₃, NO₂ oder CN substituierte Phenyl-, Phenyl-C₁₋₄-alkylen-, Naphthyl-, Naphthyl-C₁₋₆-alkylen-Gruppe, eine C₂₋₆-Alkenyl- oder C₂₋₆-Alkinylgruppe, eine C₃₋₇-Cycloalkylgruppe oder eine der Gruppen-NHR⁴, -NR⁴₂, -OR⁴, -SO₂NHR⁴, -SO₂NR⁴₂, -COR⁴ oder -CO₂R⁴ (mit R⁴ in der Bedeutung von C₁₋₄-Alkyl, Phenyl, Phenyl-C₁₋₄-alkylen, Naphthyl oder Naphthyl-C₁₋₄-alkylen) ist,
- Z²: eine der für Z¹ angegebenen Bedeutungen besitzt, jedoch kein Wasserstoffatom ist, oder
- Z¹: und Z² zusammen mit B und D auch einen der Reste 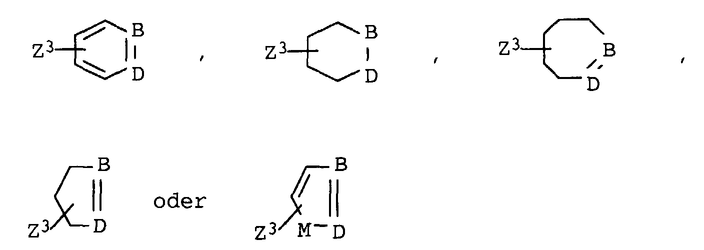 (worin Z³ eine der für Z¹ angegebenen Bedeutungen besitzt und M eine CH₂- oder NH-Gruppe ist) bedeuten,
- K: eine Alkylen- oder Alkenylgruppe mit jeweils bis zu 6 C-Atomen oder die Gruppe 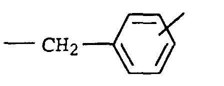 ist,
- L: 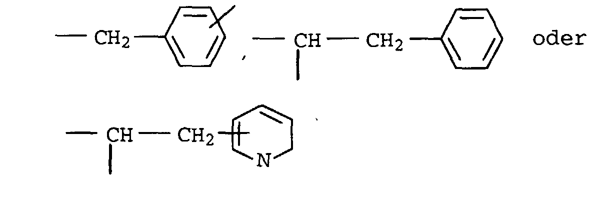 ist,
- R¹: eine -CO₂R⁹-Gruppe (mit R⁹ in der Bedeutung von Wasserstoff, C₁₋₄-Alkyl oder Benzyl), -CONR⁹₂, -OR⁹, -SR⁹, -SO₃R⁹, -PO₃R⁹₂ oder einen Tetrazolrest bedeutet und
- R²: die Gruppe 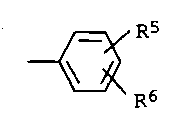 (mit R⁵ und R⁶ in der Bedeutung von Wasserstoff, C₁₋₄-Alkyl, -OR⁹ oder -SR⁹), 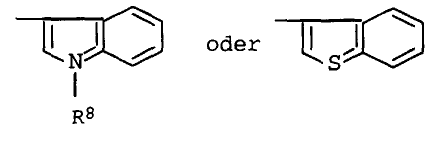 darstellt, sowie gegebenenfalls deren Salze mit physiologisch verträglichen Säuren.

Bevorzugt sind solche Verbindungen der Formeln Ia und Ib, in denen einer oder mehrere der Molekülbestandteile A, B, D, E, K, L, R¹, R², Z¹ und Z² folgende Bedeutungen besitzen:
A,B,D -CH-
E -CH-, -N-
K -CH₂-
L -(CH₂)₁₋₄-, 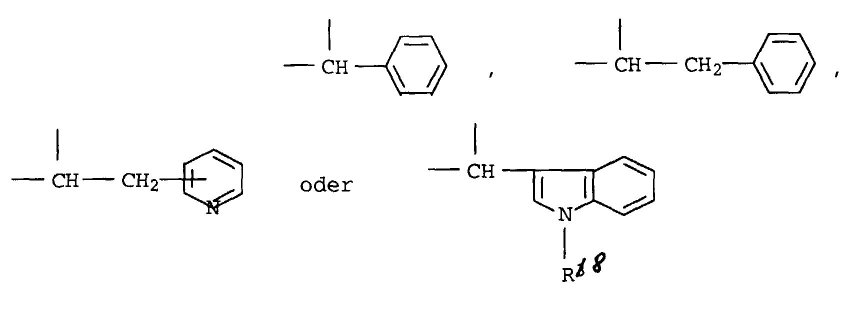
R¹ -COOH
R² 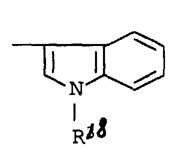 (R⁸ = H, C₁₋₃-Alkyl, -CHO, -COO-C₁₋₃-Alkyl), 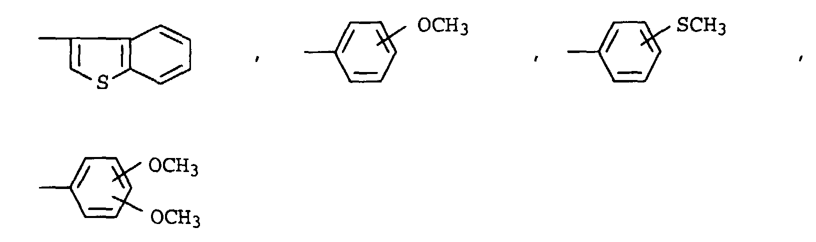
Z¹ C₁₋₆-Alkyl, C₂₋₆-Alkenyl, C₂₋₆-Alkinyl, -NH-CO-C₂₋₅-Alkyl
Z² Wasserstoff
Z¹ und Z² ein mit B und D verbundener Rest 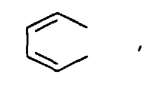 der durch einen der für Z¹ bevorzugt genannten Reste substituiert ist.

Die Verbindungen der Formel I werden - falls E ein Stickstoffatom bedeutet - auf folgendem Reaktionsweg erhalten:
Durch Umsetzung eines substituierten Aminocarbonsäurederivats der Formel II mit einem Amin der Formel III wird zunächst IV erhalten. Ringschluß mit einem aktivierten Derivat der Kohlensäure ergibt die Verbindungen der Formel V. Alkylierung des Amidstickstoffs führt zu den Verbindungen der Formel I. Die für die Durchführung der einzelnen Reaktionsschritte gegebenenfalls intermediär erforderlichen Schutzgruppen werden nach allgemein bekannten Methoden eingeführt und wieder abgespalten.

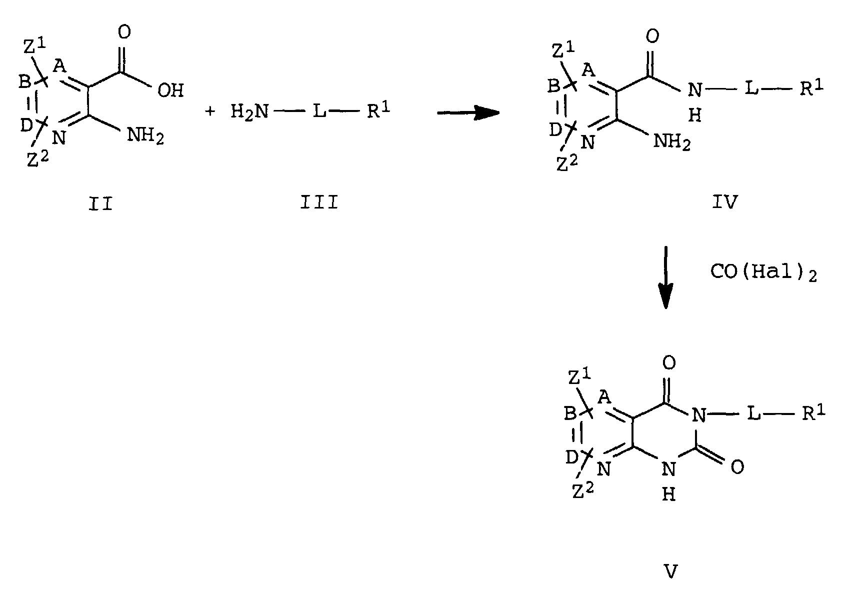

Die Verbindungen der allgemeinen Formel I werden - falls E Alkylen oder substituiertes Alkylen bedeutet - auf folgendem Weg erhalten:
Die Carbonsäurederivate der Formel IV werden durch Umsetzung mit einem aktivierten Halogencarbonsäurederivat der Formel VI zum N-Acylderivat VII umgesetzt. Als aktivierte Halogencarbonsäurederivate werden vorzugsweise die entsprechenden Säurehalogenide verwendet. Anschließend wird unter Abspaltung von Halogenwasserstoff zu VIII cyclisiert. Substitution am Amidstickstoff führt zu den Verbindungen der Formel I. Die für die Durchführung der einzelnen Reaktionsschritte intermediär erforderlichen Schutzgruppen werden nach allgemein bekannten Methoden eingeführt und wieder abgespalten. 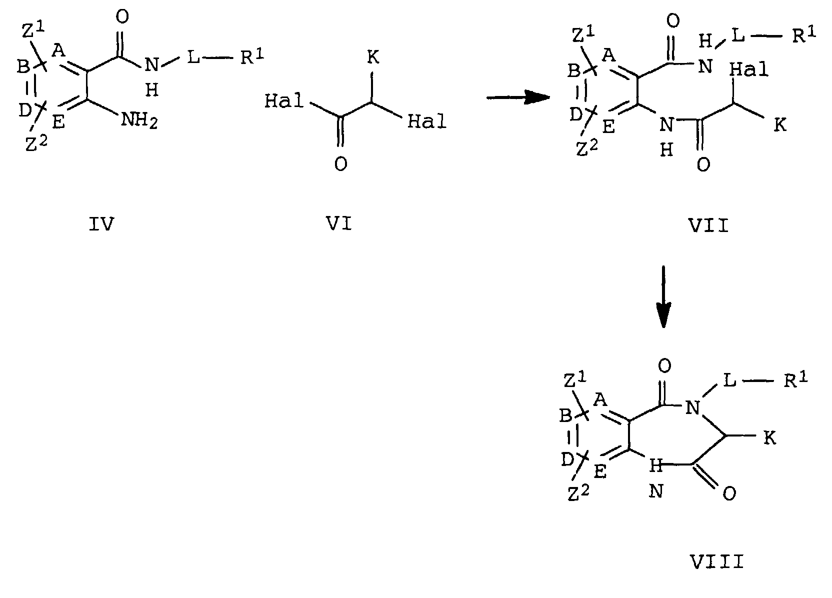
Die Verbindungen der vorliegenden Erfindung bieten ein neues therapeutisches Potential für die Behandlung von Hypertonie, pulmonaler Hochdruck, Myokardinfarkt, Angina Pectoris, akutem Nierenversagen, Niereninsuffizienz, zerebralen Vasospasmen, zerebraler Ischämie, Subarachnoidalblutungen, Migräne, Asthma, Atherosklerose, endotoxischem Schock, Endotoxin-induziertem Organversagen, intravaskulärer Koagulation, Restenose nach Angioplastie und Cyclosporin-induziertem Nierenversagen, bzw. Hypertonie.

Die gute Wirkung der Verbindungen läßt sich in folgenden Versuchen zeigen:

### Rezeptorbindungsstudien

Für Bindungsstudien wurden klonierte humane ET_{A}-Rezeptorexprimierende CHO-Zellen und Meerschweinchen-Kleinhirnmembranen mit > 60 % ET_{B}- im Vergleich zu ET_{A}-Rezeptoren eingesetzt.

### Membranpräparation

Die ET_{A}-Rezeptor-exprimierenden CHO-Zellen wurden in F₁₂-Medium mit 10 % fötalem Kälberserum, 1 % Glutamin, 100 E/ml Penicillin und 0,2 % Streptomycin (Gibco BRL, Gaithersburg, MD, USA) vermehrt. Nach 48 h wurden die Zellen mit PBS gewaschen und mit 0,05 % trypsinhaltiger PBS 5 min inkubiert. Danach wurde mit F₁₂-Medium neutralisiert und die Zeilen durch Zentrifugation bei 300 x g gesammelt. Zur Lyse der Zellen wurde kurz das Pellet mit Lysispuffer (5 mM Tris-HCl, pH 7,4 mit 10 % Glycerin) gewaschen und danach in einer Konzentration von 10⁷-Zellen/ml Lysispuffer 30 min bei 4°C inkubiert. Die Membranen wurden bei 20.000 x g 10 min zentrifugiert und das Pellet in flüssigem Stickstoff gelagert.

Meerschweinchenkleinhirne wurden im Potter-Elvejhem-Homogenisator homogenisiert und durch differentielle Zentrifugation 10 min bei 1.000 x g und wiederholte Zentrifugation des Überstandes 10 min bei 20.000 x g gewonnen.

### Bindungstests

Für den ET_{A}- und ET_{B}-Rezeptorbindungstest wurden die Membranen in Inkubationspuffer (50 mM Tris-HCl, pH 7,4 mit 5 mM MnCl₂, 40 µg/ml Bacitracin und 0,2 % BSA) in einer Konzentration von 50 µg Protein pro Testansatz suspendiert und bei 25°C mit 25 pM [125J]-ET₁ (ET_{A}-Rezeptortest) oder 25 pM [125J]-RZ₃ (ET_{B}-Rezeptortest) in Anwesenheit und Abwesenheit von Testsubstanz inkubiert. Die unspezifische Bindung wurde mit 10⁻⁷ M ET₁ bestimmt. Nach 30 min wurde der freie und der gebundene Radioligand durch Filtration über GF/B Glasfaserfilter (Whatman, England) an einem Skatron-Zellsammler (Skatron, Lier, Norwegen) getrennt und die Filter mit eiskaltem Tris-HCl-Puffer, pH 7,4 mit 0,2 % BSA gewaschen. Die auf den Filtern gesammelte Radioaktivität wurde mit einem Packard 2200 CA Flüssigkeitszintillationszähler quantifiziert.

Die Bestimmung der Kᵢ-Werte erfolgte über nichtlineare Regressionsanalyse mit dem Programm LIGAND.

### Funktionelles in vitro-Testsystem für die Suche nach Endothelinrezeptor (Subtyp A)-Antagonisten

Dieses Testsystem ist ein funktioneller, auf Zellen basierender Test für Endothelinrezeptoren. Bestimmte Zellen zeigen, wenn sie mit Endothelin 1 (ET1) stimuliert werden, einen Anstieg der intrazellulären Calciumkonzentration. Dieser Anstieg kann in intakten Zellen, die mit Calcium-sensitiven Farbstoffen beladen wurden, gemessen werden.

Aus Ratten isolierte 1-Fibroblasten, bei denen ein endogener Endothelinrezeptor vom A-Subtyp nachgewiesen wurde, wurden mit dem Fluoreszenzfarbstoff Fura 2-an wie folgt beladen: Nach Trypsinierung wurden die Zellen in Puffer A (120 mM NaCl, 5 mM KCl, 1,5 mM MgCl₂, 1 mM CaCl₂, 25 mM HEPES, 10 mM Glucose, pH 7,4) bis zu einer Dichte von 2 x 10⁶/ml resuspendiert und in 30 min bei 37°C im Dunkeln mit Fura 2-am (2 µM), Pluronics F-127 (0,04 %) und DMSO (0,2 %) inkubiert. Danach wurden die Zellen zweimal mit Puffer A gewaschen und zu 2 x 10⁶/ml resuspendiert.

Das Fluoreszenzsignal von 2 x 10⁵ Zellen pro ml bei Ex/Em 380/510 wurde bei 30°C kontinuierlich registriert. Zu den Zellen wurden die Testsubstanzen und nach einer Inkubationszeit von 3 min ET1 wurde die maximale Änderung der Fluoreszenz bestimmt. Die Antwort der Zellen auf ET1 ohne vorherige Zugabe einer Testsubstanz diente als Kontrolle und wurde gleich 100 % gesetzt.

### Testung der ET-Antagonisten in vivo

Männliche 250 - 300 g schwere SD-Ratten wurden mit Amobarbital narkotisiert, künstlich beatmet, vagotomisiert und despinalisiert. Die Arteria carotis und Vena jugularis wurden kathetisiert.

In Kontrolltieren führt die intravenöse Gabe von 1 µg/kg ET1 zu einem deutlichen Blutdruckanstieg, der über einen längeren Zeitraum anhält.

Den Testtieren wurde 5 min vor der ET1 Gabe die Testverbindungen i.v. injiziert (1 ml/kg). Zur Bestimmung der ET-antagonistischen Eigenschaften wurde der Blutdruckanstieg in den Testtieren mit dem in den Kontrolltieren verglichen.

### Endothelin-1 induzierter "sudden death" an Mäusen

Das Testprinzip besteht in der Hemmung des durch Endothelin verursachten plötzlichen Herztodes der Maus, der wahrscheinlich durch Verengung der Herzkranzgefäße bedingt ist, durch Vorbehandlung mit Endothelin-Rezeptorantagonisten. Nach intravenöser Injektion von 10 nmol/kg Endothelin im Volumen von 5 ml/kg Körpergewicht kommt es innerhalb weniger Minuten zum Tod der Tiere.

Die letale Endothelin-1 Dosis wird jeweils an einem kleinen Tierkollektiv überprüft. Wird die Prüfsubstanz intravenös appliziert, erfolgt meist 5 min danach die im Referenzkollektiv letale Endothelin-1 Injektion. Bei anderen Applikationsarten verlängern sich die Vorgabezeiten, gegebenenfalls bis zu mehreren Stunden.

Die Überlebensrate wird dokumentiert und effektive Dosen, die 50 % der Tiere 24 h oder länger gegen den Endothelin-Herztod schützen (ED 50) werden ermittelt.

### Funktioneller Gefäßtest für Endothelin-Rezeptorantagonisten

An Aortensegmenten des Kaninchens wird nach einer Vorspannung von 2 g und einer Relaxationszeit von 1 h in Krebs-Henseleitlösung bei 37°C und einem pH-Wert zwischen 7,3 und 7,4 zunächst eine K⁺-Kontraktur ausgelöst. Nach Auswaschen wird eine Endothelin-Dosiswirkungskurve bis zum Maximum erstellt.

Potentielle Endothelin-Antagonisten werden an anderen Präparaten des gleichen Gefäßes 15 min vor Beginn der Endothelin-Dosiswirkungskurve appliziert. Die Effekte des Endothelins werden in % der K⁺-Kontraktur berechnet. Bei wirksamen Endothelin-Antagonisten kommt es zur Rechtsverschiebung der Endothelin-Dosiswirkungskurve.

Die neuen Verbindungen können saure oder basische Gruppen besitzen und daher in Form von Salzen vorliegen.

Als physiologisch verträgliche Säuren kommen zur Salzbildung insbesondere in Betracht: Salzsäure, Jodwasserstoffsäure, Schwefelsäure, Phosphorsäure, Essigsäure, Zitronensäure, Malonsäure, Salicylsäure, Maleinsäure, Fumarsäure, Bernsteinsäure, Ascorbinsäure, Äpfelsäure, Methansulfonsäure, Milchsäure, Gluconsäure, Glucuronsäure, Amidosulfonsäure, Benzoesäure, Weinsäure.

Als Basen eignen sich beispielsweise Alkali- und Erdalkalimetallhydroxide.

Die erfindungsgemäßen Verbindungen können in üblicher Weise oral oder parenteral (subkutan, intravenös, intramuskulär, intraperitoneal) verabfolgt werden. Die Applikation kann auch mit Dämpfen oder Sprays durch den Nasen-Rachenraum erfolgen.

Die Dosierung hängt vom Alter, Zustand und Gewicht des Patienten sowie von der Applikationsart ab. In der Regel beträgt die tägliche Wirkstoffdosis zwischen etwa 0,5 und 50 mg/kg Körpergewicht bei oraler Gabe und zwischen etwa 0,1 und 10 mg/kg Körpergewicht bei parenteraler Gabe.

Die neuen Verbindungen können in den gebräuchlichen galenischen Applikationsformen fest oder flüssig angewendet werden, z.B. als Tabletten, Filmtabletten, Kapseln, Pulver, Granulate, Dragees, Suppositorien, Lösungen, Salben, Cremes oder Sprays. Diese werden in üblicher Weise hergestellt. Die Wirkstoffe können dabei mit den üblichen galenischen Hilfsmitteln wie Tablettenbindern, Füllstoffen, Konservierungsmitteln, Tablettensprengmitteln, Fließreguliermitteln, Weichmachern, Netzmitteln, Dispergiermitteln, Emulgatoren, Lösungsmitteln, Retardierungsmitteln, Antioxidantien und/oder Treibgasen verarbeitet werden (vgl. H. Sucker et al.: Pharmazeutische Technologie, Thieme-Verlag, Stuttgart, 1991). Die so erhaltenen Applikationsformen enthalten den Wirkstoff normalerweise in einer Menge von 0,1 bis 90 Gew.-%.

### Beispiel 1

### a) 5-Iod-Isatosäureanhydrid

10 g (0,038 mmol) 5-Iod-anthranilsäure wurden in 180 ml THF gelöst und 3,76 g (0,0127 mol) Bis-(trichlormethylcarbonat), gelöst in 20 ml THF, zugegeben. Man ließ noch 1 h bei Raumtemperatur und 2 h bei 50°C rühren. Nach Abkühlen wurde der Niederschlag abgesaugt und getrocknet. Man erhielt 7,8 g (71 %) 5-Iod-isatosäureanhydrid als weiße Kristalle.

### b) 2-Amino-5-iodbenzoesäure-(2-carboxylmethylethyl)-amid

7,28 g (25,2 mmol) 5-Iod-isatosäureanhydrid und 3,52 g (25,2 mmol) β-Alaninmethylester-hydrochlorid wurden in 60 ml DMF vorgelegt und 6,12 g (60,5 mmol) Triethylamin zugetropft. Die Mischung wurde 4 h auf 50°C erhitzt, abgekühlt und auf eine Mischung von 15 ml 2N NaOH mit Eis gegeben. Die wäßrige Phase wurde mit Essigester extrahiert, getrocknet und im Vakuum eingeengt. Man erhielt 6,6 g eines gelben Öls, das über Kieselgel mit Essigester/n-Heptan (1:1) chromatographisch gereinigt wurde. Man erhielt 4,6 g (52 %) 2-Amino-5-iodbenzoesäure-(2-carboxymethylethyl)amid mit einem R_{F}-Wert von 0,40 (Essigester/n-Heptan, 2:1).

### c) 6-Iod-3-(2-Carboxymethylethyl)-chinazolin-2,4-dion

4,6 g 2-Amino-5-iodbenzoesäure-(2-carboxymethylethyl)-amid wurden mit 2,66 g (26,3 mmol) Triethylamin in 50 ml Dichlormethan vorgelegt und unter Stickstoff 1,21 g (4,1 mmol) Bis-(trichlormethyl)-carbonat, gelöst in 15 ml Dichlormethan, zugetropft. Die Mischung wurde 2 h unter Rückfluß erhitzt und nach Abkühlen auf Eiswasser gegeben. Die wäßrige Phase wurde mit Dichlormethan extrahiert, getrocknet und eingeengt. Man erhielt 3,64 g (74 %) 6-Iod-3-(2-carboxymethylethyl)-chinazolin-2,4-dion [R_{F} 0,52 (Essigester/n-Heptan, 2:1)] als gelblichen Feststoff.

### d) 6-Iod-3-(2-carboxymethylethyl)-1-[3-(N-t-butylcarbamoyl)-indolylmethyl]-chinazolin-2,4-dion

2,6 g (7 mmol) 6-Iod-3-(2-carboxymethylethyl)-chinazolin-2,4-dion und 1,95 g (14,1 mmol) Kaliumcarbonat wurden in Aceton suspendiert. Zu dieser Mischung gab man 3,05 g (9,8 mmol) N-t-Butylcarbamoyl-3-indolylmethylbromid, in 20 ml Aceton gelöst, und rührte 5 h bei Raumtemperatur. Danach wurde das Solvens im Vakuum abgezogen, und der Rückstand in 50 ml Phosphatpuffer (pH 7) und 150 ml Essigester aufgenommen. Das Produkt wurde mit Essigester extrahiert, getrocknet und im Vakuum eingeengt. Man erhielt 5,9 g dunkles Öl, das über Kieselgel mit Essigester/n-Heptan (Verhältnis 1:4) als Eluent chromatographiert wurde. Man erhielt 1,9 g (45 %) 6-Iod-3-(2-carboxymethylethyl)-1-[3-(N-t-butylcarbamoyl)-indolylmethyl]-chinazolin-2,4-dion, R_{F} 0,18 (Essigester/n-Heptan, 1:4).

### e) 6-(Pent-1-enyl)-3-(2-carboxymethylethyl)-1-[3-(N-t-butyl-carbamoyl)-indolylmethyl]-chinazolin-2,4-dion

1,09 g (1,8 mmol) 6-Iod-3-(2-carboxymethylethyl)-1-[3-(N-t-butylcarbamoyl)-indolylmethyl]-chinazolin-2,4-dion und 0,75 g (5m4 mmol) Kaliumcarbonat wurden in 15 ml DMF vorgelegt und 0,63 g (9,0 mmol) 1-Penten, 0,58 g (1,8 mmol) Tetrabutylammoniumbromid und 10 mg Palladiumacetat zugegeben. Die Mischung wurde 48 g bei Raumtemperatur gerührt und danach im Vakuum eingeengt. Das Rohprodukt wurde über Kieselgel chromatographiert, Eluent Dichlormethan (2 % Methanol). Man erhielt 0,69 g (70 %) Produkt, R_{F} 0,12 (Dichlormethan + 2 % Methanol).

### f) 6-(Pent-1-enyl)-3-(2-carboxymethylethyl)-1-(3-indolylmethyl)-chinazolin-2,4-dion

0,53 g des gemäß e) erhaltenen Produkts wurden in 5 ml Dichlormethan gelöst, 1,13 g (9,9 mol) Trifluoressigsäure zugegeben und 16 h bei Raumtemperatur gerührt. Anschließend wurde mit Wasser gewaschen, getrocknet und eingeengt. Man erhielt 0,45 g Rohprodukt, das ohne Aufreinigung weiter umgesetzt wurde.

### g) 6-(Pent-1-enyl)-3-(2-carboxylethyl)-1-(3-indolylmethyl)-chinazolin-2,4-dion

0,45 g des gemäß f) erhaltenen Rohprodukts wurden in 10 ml THF gelöst und 0,03 mg (1,25 mmol) Lithiumhydroxid, in 2 ml Wasser gelöst, zugegeben und bei Raumtemperatur 16 h gerührt. Das Solvens wurde im Vakuum abgezogen, der Rückstand mit Essigester und Wasser aufgenommen; die wäßrige Phase mit Ammoniak-Lösung auf pH 9 gebracht und mit Essigester extrahiert. Die organische Phase wurde getrocknet und eingeengt. Man erhielt 0,37 g Rohprodukt. Nach HPLC (reversed-phase-Material, Acetonitril/Wasser) erhielt man 0,12 g (0,28 mmol) 6-(Pent-1-enyl)-3-(2-carboxy-ethyl)-1-(3-indolylmethyl)-chinazolin-2,4-dion.

Analog Beispiel 1 wurden hergestellt:
6-[E-(3-Methylbut-1-en-yl)]-3-(2-carboxyl-ethyl)-1-(3-indolylmethyl)-chinazolin-2,4-dion
6-[E-(2-[p-(1,1-Dimethylethyl)-phenyl)]-ethenyl-3-(2-carboxylethyl)-1-(3-indolylmethyl)chinazolin-2,4-dion
6-[E-(4-Methylpent-1-en-yl)]-3-(2-carboxyl-ethyl)-1-(3-indolylmethyl)-chinazolin-2,4-dion
6-[E-(2-Cyclohexyl-ethenyl)]-3-(2-carboxyl-ethyl)-1-(3-indolylmethyl)-chinazolin-2,4-dion, Schmp. 122-126°C
6-[E-(Pent-1-enyl)]-3-(carboxyl-methyl)-1-(3-[N-methylindolyl)-methyl]-chinazolin-2,4-dion, Schmp. > 300°C
6-[E-(4-Methylpent-1-enyl)]-3-(carboxyl-methyl)-1-[3-(N-methylindolylmethyl)]-chinazolin-2,4-dion, Schmp. > 300°C
6-[E-(3,3-Dimethylbut-1-enyl)]-3-(carboxyl-methyl)-1-[3-(N-methylindolylmethyl)]-chinazolin-2,4-dion, Schmp. > 300°C

### Beispiel 2

### a) 6-Iod-3-(carboxymethyl-methyl)-1-[3-(N-methylindolyl)]-chinazolin-2,4-dion

12,0 g (33,3 mmol) des analog Beispiel 1a)-c) hergestellten 6-Iod-3-(carboxymethyl-methyl)-chinazolin-2,4-dions wurden in 250 ml DMF suspendiert und 0,2 g (66,6 mmol) Kaliumcarbonat zugegeben. Danach wurden 16,5 g (49,9 mmol) [3-(N-Methylindolyl)-methyl]-trimethylammoniumiodid zugegeben und 6 h unter Rückfluß erhitzt. Nach Abziehen des Lösungsmittels im Vakuum wurde mit Wasser und Essigester versetzt und das Produkt abgesaugt und getrocknet. Man erhielt 16,2 g (96,5 %) Produkt, R_{F} 0,6 (Essigester/n-Heptan, 2:1).

### b) 6-(Pent-1-enyl)-3-(carboxymethyl-methyl)-1-[3-(N-methylindolyl)]-chinazolin-2,4-dion

2,5 g (4,97 mmol) des gemäß a) erhaltenen Produkts wurden in 20 ml DMF vorgelegt und 1,46 g (14,9 mmol) Kaliumacetat, 1,74 g (24,8 mmol) 1-Penten, 1,6 g (4,97 mmol) Tetrabutylammoniumbromid sowie 28 mg Palladium(II)-acetat zugegeben. Die Mischung wurde 16 h bei Raumtemperatur gerührt. Das Solvens wurde im Vakuum abgezogen und der Rückstand in Essigester und 10 %iger EDTA-Lösung aufgenommen. Die organische Phase wurde nochmal mit EDTA-Lösung gewaschen, getrocknet und eingeengt. Der dunkle, ölige Rückstand wurde über Kieselgel mit Essigester/n-Heptan (1:5) als Eluent chromatographiert. Man erhielt 1,23 g (55,5 %) Produkt, R_{F} 0,61 (Essigester/ n-Heptan, 1:1).

### c) 6-Pentyl-3-(carboxymethyl-methyl)-1-[3-(N-methylindolyl)]-chinazolin-2,4-dion

0,2 g (0,45 mmol) des gemäß b) erhaltenen Produkts wurden in Essigester gelöst. Nach Zugabe von 0,1 g Palladium-Aktivkohle (10 % Pd) wurde unter Wasserstoffatmosphäre 6 h gerührt, wobei 15 ml Wasserstoff verbraucht wurden. Nach Abfiltrieren des Katalysators über Kieselgel wurde mit Wasser gewaschen, getrocknet und eingeengt. Man erhielt 0,2 g eines hellgelben Feststoffs, der als Rohprodukt weiterverarbeitet wurde.

### d) 6-Pentyl-3-(carboxyl-methyl)-1-[3-(N-methylindolyl)]-chinazolin-2,4-dion

0,2 g (0,45 mmol) des gemäß c) erhaltenen Produkts wurden nach der Vorschrift des Beispiels 1 g mit Lithiumhydroxid hydrolysiert. Man erhielt 0,11 g Produkt, R_{F} 0,40 (Dichlormethan, 20 % Methanol).

### Beispiel 3

### a) 6-(2-Phenylethinyl)-3-(carboxylmethyl-methyl)-1-[3-(N-methylindolyl)]-chinazolin-2,4-dion

3,5 g (6,95 mmol) 6-Iod-3-(carboxymethyl-methyl)-1-[3-(N-methylindolyl)]-chinazolin-2,4-dion wurden in 50 ml Essigester gelöst und 1,42 g (13,9 mmol) Phenylacetylen, 0,49 g (0,65 mmol) Bis(triphenylphosphin)palladiumdichlorid, 0,066 g Kupfer(I)-iodid und 3,52 g (34,8 mmol) Triethylamin zugegeben. Die Reaktionsmischung wurde 8 h unter Rückfluß erhitzt. Danach wurden 50 ml Essigester zugegeben. Nach Waschen mit 10 %iger EDTA-Lösung wurde getrocknet und eingeengt. Der braune, feste Rückstand wurde mit n-Heptan/Essigester (4:1) als Eluent über Kieselgel chromatographiert. Man erhielt 2,76 g (86 %) Produkt, R_{F} 0,19 (n-Heptan/Essigester, 2:1).

### b) 6-(2-Phenylethinyl)-3-(carboxylmethyl)-1-[3-(N-methylindolyl)]-chinazolin-2,4-dion

1,5 g (3,25 mmol) des gemäß a) erhaltenen Produkts wurden mit 0,12 g (5,0 mmol) Lithiumhydroxid analog Beispiel 1g) umgesetzt. Das in Wasser und Essigester unlösliche Produkt wurde abgesaugt und getrocknet. Man erhielt 0,78 g (52 %) Produkt, R_{F} 0,72 (n-Heptan/Essigester, 1:2), Schmp. > 300°C.

Analog wurde hergestellt:
6-(Pent-1-inyl)-3-(carboxylmethyl)-1-[3-(N-methylindolyl)]-chinazolin-2,4-dion

Analog können hergestellt werden:
6-(4-Methylpent-1-inyl)-3-(carboxylmethyl)-1-[3-(N-methylindolyl)]-chinazolin-2,4-dion
6-(4-Methylpent-1-inyl)-3-2-carboxyl-ethyl)-1-[3-(N-methylindolyl)]-chinazolin-2,4-dion
6-(4-Methylpent-1-inyl)-3-(3-carboxylpropyl)-1-[3-(N-methylindolyl)]-chinazolin-2,4-dion

### Beispiel 4

### a) 3-(2-Methylpropoxy)-6-nitrobenzaldehyd

50 g (0,308 mmol) 3-Hydroxy-6-nitrobenzaldehyd und 49,3 g (0,36 mol) 2-Methylpropylbromid wurden in 300 ml DMF gelöst und 45,5 g (0,33 mol) Kaliumcarbonat zugegeben. Die Mischung wurde 5 h unter Rückfluß erhitzt. Nach dem Abkühlen wurde das Solvens im Vakuum abgezogen, der Rückstand in Essigester gelöst und mit 10 %iger Natriumcarbonat-Lösung und Kochsalz-Lösung gewaschen. Nach dem Trocknen und Einengen wurde der Rückstand über Kieselgel mit n-Heptan/Essigester (9:1) chromatographiert. Man erhielt 55,2 g (80 %) orangefarbene Kristalle, R_{F} 0,43 (n-Heptan/Essigester, 9:1).

### b) 3-(2-Methylpropoxy)-6-nitrobenzoesäure

24,2 g (0,067 mmol) t-Butylammoniumpermanganat, gelöst in 100 ml Pyridin, wurden unter Kühlung so zur Lösung von 22,3 g (0,10 mmol) des gemäß a) erhaltenen Produkts in 150 ml Pyridin zugetropft, daß die Temperatur nicht über 20°C stieg. Die Mischung wurde 16 h bei Raumtemperatur gerührt, dann auf eine Eis-Salzsäure-Mischung gegossen und mit Na₂S₂O₃ entfärbt. Die wäßrige Phase wurde mit Essigester extrahiert, die organische Phase mit Ammoniak alkalisch gemacht und das Produkt in die wäßrige Phase extrahiert. Nach Ansäuern der Wasser-Phase wurde das Produkt wieder mit Essigester extrahiert, getrocknet und eingeengt. Das Produkt wurde aus Dichlormethan umkristallisiert. Man erhielt 12,0 g (75 %) 3-(2-Methylpropoxy)-6-nitrobenzoesäure.

### c) 3-(2-Methylpropoxy)-6-aminobenzoesäure

8,8 g (36,8 mmol des gemäß b) erhaltenen Produkts wurde in 50 ml Eisessig gelöst. 1,5 g Palladium-Aktivkohle (10 %) wurden 5 h unter Wasserstoff-Atmosphäre gerührt. Der Katalysator wurde über Kieselgel abgesaugt und das Solvens im Vakuum abgezogen. Der Rückstand wurde mit Wasser versetzt und das Produkt abgesaugt, mit Ether gewaschen und getrocknet. Man erhielt 6,87 g (89 %) Feststoff, R_{F} 0,3 (n-Heptan/Essigester, 1:1).

### d) 3-(2-Methylpropoxy)-isatosäureanhydrid

6,87 g (32,8 mmol) des gemäß c) erhaltenen Produkts wurde analog Beispiel 1a) zum Isatosäureanhydrid umgesetzt. Man erhielt 6,51 g (84 %) Rohprodukt, das ohne Aufreinigung weiterverarbeitet wurde.

### e) 2-Amino-5-(2-Methylpropoxy)-benzoesäure-(2-carboxymethyl-ethyl)-amid

6,51 g (27,7 mmol) des gemäß d) erhaltenen Produkts wurden analog Beispiel 1b) mit β-Alaninmethylester-hydrochlorid umgesetzt.
   Das Rohprodukt wurde mit n-Heptan/Essigester (2:1) über Kieselgel chromatographiert. Man erhielt 5,21 g (64 %) Produkt, R_{F} 0,2 (n-Heptan/Essigester, 1:1).

### f) 6-(2-Methylpropoxy)-3-(-2-carboxymethyl-ethyl)-chinazolin-2,4-dion

5,21 g (17,7 mmol) des gemäß e) erhaltenen Produkts wurden analog Beispiel 1c) umgesetzt. Das Rohprodukt wurde mit n-Heptan/Essigester (2:1) über Kieselgel chromatographiert. Man erhielt 3,28 g (58 %) Produkt, R_{F} 0,28 (n-Heptan/Essigester, 1:1).

### g) 6-(2-Methylpropoxy)-3-(2-carboxymethyl-ethyl)-1-(3-indolylmethyl)-chinazolin-2,4-dion

0,35 g (8,76 mmol) 60 %iges Natriumhydrid wurden in 40 ml THF suspendiert und 2,34 g (7,3 mmol) des gemäß f) erhaltenen Produkts in 30 ml DMF gelöst zugetropft. Nach 30 min wurden 2,77 g (8,76 mmol) frisch hergestelltes 3-(Indolylmethyl)-trimethylammoniumiodid, in 40 ml DMF gelöst, zugetropft und 18 h bei Raumtemperatur gerührt. Nach Abziehen des Solvens im Vakuum wurde der Rückstand in Essigester aufgenommen und mit 5 %iger Zitronensäure-Lösung und Wasser gewaschen. Nach Trocknen und Einengen wurde das Rohprodukt mit n-Heptan/ Essigester (2:1) über Kieselgel chromatographiert. Man erhielt 1,29 g (39 %) Produkt, R_{F} 0,52 (n-Heptan/Essigester, 1:1).

### h) 6-(2-Methylpropoxy)-3-(2-carboxyl-ethyl)-1-(3-indolylmethyl)-chinazolin-2,4-dion

1,23 g (2,7 mmol) des gemäß g) erhaltenen Produkts wurden nach der Vorschrift des Beispiels 1f) mit Lithiumhydroxid hydrolysiert. Das Rohprodukt wurde mit Acetonitril/Wasser über HPLC gereinigt.
   Man erhielt 0,56 g (48 %) Produkt, Schmp. 199-201°C).

Analog wurden hergestellt:
6-(2-Methylpropoxy)-3-[3-(indolyl)ethyl)-1-(carboxyl-methyl)-chinazolin-2,4-dion
6-(2-Methylpropoxy)-3-[3-(indolyl)ethyl]-1-(2-carboxyl-ethyl)-chinazolin-2,4-dion
6-(2-Methylpropoxy)-3-[3-(indolyl)ethyl]-1-(3-carboxyl-propyl)-chinazolin-2,4-dion
6-(3-Methylbutoxy)-3-(2-carboxyl-ethyl)-1-(3-indolyl-methyl)-chinazolin-2,4-dion

Aus den entsprechenden 4- bzw. 6-Iod-isatosäureanhydriden lassen sich analog Beispiel 1-3 herstellen:
5-(Pent-1-enyl)-3-(2-carboxyl-ethyl)-1-[3-(N-methyl-indolyl-methyl)]-chinazolin-2,4-dion
5-(Pentyl)-3-(2-carboxyl-ethyl)-1-[3-(N-methyl-indolyl-methyl)]-chinazolin-2,4-dion
5-(4-Methyl-pent-1-enyl)-3-(carboxyl-ethyl)-1-[3-(N-methyl-indolyl-methyl)]-chinazolin-2,4-dion
5-(4-Methyl-pentyl)-3-(carboxyl-ethyl)-1-[3-(N-methyl-indolyl-methyl)]-chinazolin-2,4-dion
5-(3-Methyl-but-1-enyl)-3-(carboxyl-ethyl)-1-[3-(N-methyl-indolyl-methyl)]-chinazolin-2,4-dion
5-(3-Methyl-butyl)-3-(carboxyl-ethyl)-1-[3-(N-methyl-indolyl-methyl)]-chinazolin-2,4-dion
7-(Pent-1-enyl)-3-[2-carboxyl-ethyl-1-(3-(N-methyl-indolyl-methyl)]-chinazolin-2,4-dion
7-(Pentyl)-3-(2-carboxyl-ethyl)-1-[3-(N-methyl-indolyl-methyl)]-chinazolin-2,4-dion
7-(4-Methylpent-1-enyl)-3-(carboxyl-ethyl)-1-[3-(N-methyl-indolyl-methyl)]-chinazolin-2,4-dion
7-(3-Methyl-but-1-enyl)-3-(carboxyl-ethyl)-1-[3-(N-methyl-indolyl-methyl)]-chinazolin-2,4-dion
7-(3-Methyl-butyl)-3-(carboxyl-ethyl)-1-[3-(N-methyl-indolyl-methyl)]-chinazolin-2,4-dion

### Beispiel 5

### a) 3-Iod-6-(bromacetyl-amino)-benzoesäure-(2-carboxy-methyl-ethyl)-amid

21,0 g (60,5 mmol) des nach Beispiel 1b) hergestellten 3-Iod-6-amino-benzoesäure-(2-carboxy-methyl-ethyl)amids wurden in 270 ml Dichlormethan gelöst und 12,5 ml (90,7 mmol) Triethylamin zugesetzt. Bei -30°C wurden 7,8 ml (90,7 mmol) Bromessigsäurebromid in 80 ml Dichlormethan zugetropft. Die Mischung wurde noch 2 h beim Raumtemperatur gerührt, dann mit 10 %iger Zitronensäure-Lösung und Natriumhydrogencarbonat-Lösung gewaschen, getrocknet und eingeengt. Das Produkt wurde aus Dichlormethan umkristallisiert, man erhielt 26,1 g (92 %) hellgelbe Kristalle.

### b) 7-Iod-4-(carboxymethyl-ethyl)-benzo-1,4-diazepin-3,5-dion

7,5 g (16 mmol) des gemäß a) erhaltenen Produkts wurden in 100 ml Methanol suspendiert und langsam zur Lösung von 32 mmol Natriummethanolat in 400 ml Methanol zugetropft. Die Mischung wurde 20 h gerührt, danach auf Phosphatpuffer (pH 7) gegossen, das Methanol im Vakuum abgezogen und die wäßrige Phase mit Essigester extrahiert, getrocknet und eingeengt. Das Rohprodukt wurde mit Dichlormethan (+ 3 % Methanol) über Kieselgel chromatographiert. Man erhielt 2,6 g (41 %) Produkt, R_{F} 0,49 (Dichlormethan, 7 % Methanol).

### c) 7-Iod-1-[3-(N-methyl-indolyl)-methyl]-4-(2-carboxymethylethyl)-benzo-1,4-diazepin-3,5-dion

2,6 g (6,6 mmol) des gemäß b) erhaltenen Produkts wurden in 100 ml DMF gelöst, 1,82 g (1,32 mmol) Kaliumcarbonat zugegeben und danach 3,27 g (9,9 mmol) 3-(N-Methyl-indolyl)-methyltrimethylammoniumiodid zugesetzt. Die Reaktionsmischung wurde 6 h unter Rückfluß erhitzt, danach das DMF im Vakuum abgezogen und der Rückstand in Wasser aufgenommen. Die wäßrige Phase wurde mit Essigester extrahiert, getrocknet und eingeengt. Das Rohprodukt wurde mit Essigester/n-Heptan (1:1) als Eluent über Kieselgel chromatographiert .Man erhielt 2,1 g (59 %) Produkt, R_{F} 0,57 (Dichlormethan, 7 % Methanol).

### d) 7-(Pent-1-enyl)-1-[3-(N-methylindolyl)-methyl]-4-(carboxy-methyl-ethyl)-benzo-1,4-diazepin-3,5-dion

2,1 g (3,9 mmol) des gemäß c) erhaltenen Produkts und 1,63 g (11,7 mmol) Kaliumcarbonat wurden in 40 ml DMF vorgelegt und 1,37 g (19,5 mmol) 1-Penten, 1,26 g (3,9 mmol) Tetrabutylammoniumbromid und 20 g Palladium-II-acetat zugegeben. Die Mischung wurde 2 h bei 50°C und 16 h bei Raumtemperatur gerührt. Danach wurde das Solvens im Vakuum abgezogen und der Rückstand mit Dichlormethan (+ 2 % Methanol) über Kieselgel chromatographiert. Man erhielt 1,55 g (3,3 mmol = 84 %) Produkt, R_{F} 0,65 (Dichlormethan, 7 % Methanol).

### e) 7-(Pent-1-enyl)-1-[3-(N-methylindolyl)-methyl]-4-(carboxyl-ethyl)-benzo-1,4-diazepin-3,5-dion

0,55 g (1,16 mmol) des gemäß d) erhaltenen Produkts wurden nach der Vorschrift des Beispiels 1g) hydrolysiert. Das Rohprodukt wurde über HPLC (reversed-phase-Material, Acetonitril/Wasser) aufgereinigt.
   Man erhielt 0,22 g (0,48 mmol = 41,4 %) Produkt.

Analog können hergestellt werden:
7-(4-Methylpent-1-enyl)-1-[3-(N-methylindolyl)-methyl]-4-(2-carboxyl-ethyl)-benzo-1,4-diazepin-3,5-dion
7-(3-Methylbut-1-enyl)-1-[3-(N-methylindolyl)-methyl]-4-(2-carboxyl-ethyl)-benzo-1,4-diazepin-3,5-dion
7-(4-Methylpent-1-enyl)-1-[3-(N-methylindolyl)-methyl]-4-(2-carboxyl-methyl)-benzo-1,4-diazepin-3,5-dion
6-(3-Methylbut-1-enyl)-1-[3-(N-methylindolyl)-methyl]-4-(2-carboxyl-ethyl)-benzo-1,4-diazepin-3,5-dion
8-(3-Methylbut-1-enyl)-1-[3-(N-methylindolyl)-methyl]-4-(2-carboxyl-ethyl)-benzo-1,4-diazepin-3,5-dion

### Beispiel 6

### a) 7-(Pentyl)-1-[3-(N-methylindolyl)-methyl]-4-(2-carboxy-methyl)-benzo-1,4-diazepin-3,5-dion

1,0 g (2,1 mmol) der Substanz des Beispiels 5d) wurden analog Beispiel 2b) hydriert. Nach Chromatographie über Kieselgel mit Essigester/n-Heptan (1:4) erhielt man 0,63 g (63 %) Produkt, R_{F} 0,53 (Essigester/n-Heptan, 1:1).

### b) 7-(Pentyl)-1-[3-(N-methylindolyl)-methyl]-4-(2-carboxyl-ethyl)-benzo-1,4-diazepin-3,5-dion

0,5 g (1,05 mmol) des gemäß c) erhaltenen Produkts wurden analog Beispiel 1g) hydrolysiert. Das Rohprodukt wurde über HPLC (reversed-phase-Material/Acetonitril/Wasser) aufgereinigt. Man erhielt 0,20 g (0,43 mmol = 41 %) Produkt.

Analog können hergestellt werden:
7-(3-Methylbutyl)-1-[3-(N-methylindolyl)-methyl]-4-(2-carboxyl-ethyl-benzo-1,4-diazepin-3,5-dion
7-(3-Methylbutyl)-1-[3-(N-methylidolyl)-methyl]-4-(3-carboxyl-propyl-benzo-1,4-diazepin-3,5-dion
6-(3-Methylbutyl)-1-[3-(N-methylindolyl)-methyl]-4-(2-carboxyl-ethyl-benzo-1,4-diazepin-3,5-dion
8-(3-Methylbutyl)-1-[3-(N-methylindolyl)-methyl]-4-(2-carboxyl-ethyl-benzo-1,4-diazepin-3,5-dion

## Patentansprüche

1. Verwendung von Bicyclen-Derivaten der Formel I 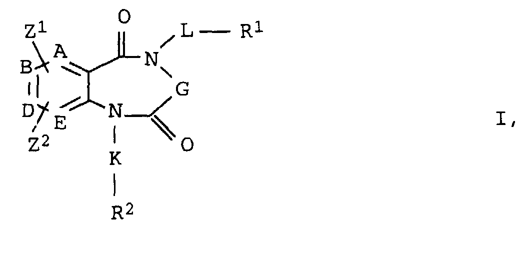 worin
2 der Reste A, B, D, E CH-Gruppen und die 2 anderen Reste CH-Gruppen oder Stickstoffatome bedeuten,
Z¹ ein Wasserstoff- oder Halogenatom, eine C₁₋₆-Alkylgruppe, eine gegebenenfalls am aromatischen Rest durch C₁-₆-Alkyl, C₁-₆-Alkoxy, Halogen, CF₃, NO₂ oder CN substituierte Phenyl-, Phenyl-C₁₋₄-alkylen-, Naphthyl-, Naphthyl-C₁₋₆-alkylen-Gruppe, eine C₂₋₆-Alkenyl- oder C₂₋₆-Alkinylgruppe, eine C₃₋₇-Cycloalkylgruppe oder eine der Gruppen-NHR⁴, -NR⁴₂, -OR⁴, -SO₂NHR⁴, -SO₂NR⁴₂, -COR⁴ oder -CO₂R⁴ (mit R⁴ in der Bedeutung von C₁₋₄-Alkyl, Phenyl, Phenyl-C₁₋₄-alkylen, Naphthyl oder Naphthyl-C₁₋₄-alkylen) ist,
Z² eine der für Z¹ angegebenen Bedeutungen besitzt, jedoch kein Wasserstoffatom ist, oder
Z¹ und Z² zusammen mit B und D auch einen der Reste 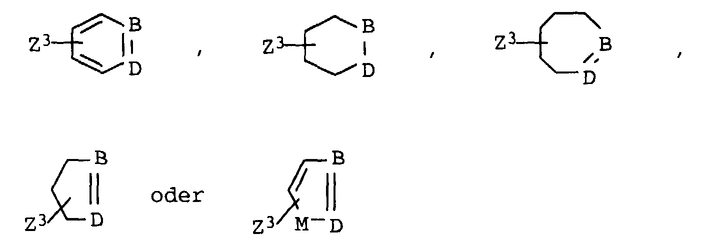 (worin Z³ eine der für Z¹ angegebenen Bedeutungen besitzt und M eine CH₂- oder NH-Gruppe ist) bedeuten,
G eine direkte Bindung oder die Gruppe CH-K¹ darstellt (mit K¹ in der Bedeutung von Wasserstoff, C₁₋₆-Alkyl, einer gegebenenfalls im Arylteil durch C₁₋₄-Alkyl, C₁₋₄-Alkoxy, Halogen, CF₃, NO₂ oder CN substituierten Phenyl-, Benzyl-, Naphthyl- oder Naphthylmethylen-Gruppe),
K eine Alkylen- oder Alkenylgruppe mit jeweils bis zu 6 C-Atomen oder die Gruppe 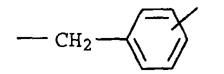 ist,
L eine Alkylen-, Alkenylen- oder Alkinylengruppe mit jeweils bis zu 6 C-Atomen oder eine der Gruppen 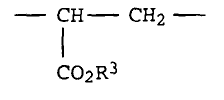 (mit R³ in der Bedeutung eines Wasserstoffatoms, einer C₁₋₄-Alkyl-, Benzyl- oder Naphthylmethylengruppe), 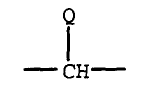 [mit Q in der Bedeutung von C₁-C₆-Alkyl, Aryl oder CH₂-R⁷, worin R⁷ Phenyl, 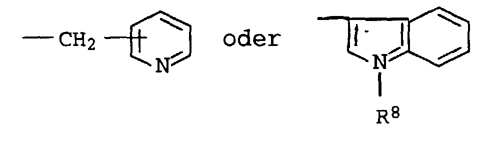 (R⁸= H, C₁₋₃-Alkyl, -CHO, -COO-C₁₋₃-Alkyl) bedeutet] oder 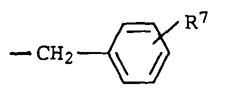
R¹ eine -CO₂R⁹-Gruppe (mit R⁹ in der Bedeutung von Wasserstoff, C₁₋₄-Alkyl oder Benzyl), -CONR⁹₂, -OR⁹, -SR⁹, -SO₃R⁹, -PO₃R⁹₂ oder einen Tetrazolrest und
R² die Gruppe 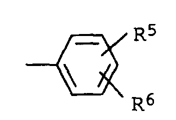 (mit R⁵ und R⁶ in der Bedeutung von Wasserstoff, C₁₋₄-Alkyl, -OR⁹ oder -SR⁹), 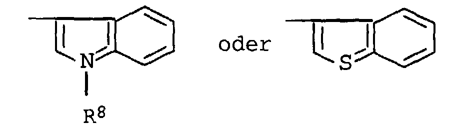
darstellen, sowie gegebenenfalls deren Salzen mit physiologisch verträglichen Säuren zur Herstellung von Arzneimitteln zur Bekämpfung von Krankheiten, die auf einem erhöhten Endothelinspiegel beruhen.

2. Bicyclen-Derivate der Formel I 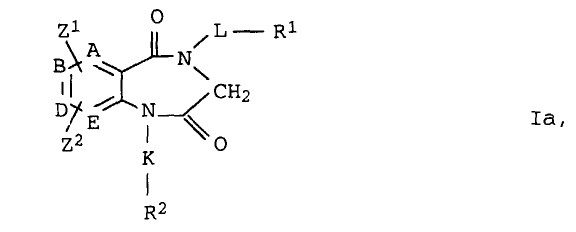 worin
2 der Reste A, B, D, E CH-Gruppen und die 2 anderen Reste CH-Gruppen oder Stickstoffatome bedeuten,
Z¹ ein Wasserstoff- oder Halogenatom, eine C₁₋₆-Alkyl-gruppe, eine gegebenenfalls am aromatischen Rest durch C₁₋₆-Alkyl, C₁₋₆-Alkoxy, Halogen, CF₃, NO₂ oder CN substituierte Phenyl-, Phenyl-C₁₋₄-alkylen-, Naphthyl-, Naphthyl-C₁₋₆-alkylen-Gruppe, eine C₂₋₆-Alkenyl- oder C₂₋₆-Alkinylgruppe, eine C₃₋₇-Cycloalkylgruppe oder eine der Gruppen-NHR⁴, -NR⁴₂, -OR⁴, -SO₂NHR⁴, -SO₂NR⁴₂, -COR⁴ oder -CO₂R⁴ (mit R⁴ in der Bedeutung von C₁₋₄-Alkyl, Phenyl, Phenyl-C₁₋₄-alkylen, Naphthyl oder Naphthyl-C₁₋₄-alkylen) ist,
Z² eine der für Z¹ angegebenen Bedeutungen besitzt, jedoch kein Wasserstoffatom ist, oder
Z¹ und Z² zusammen mit B und D auch einen der Reste 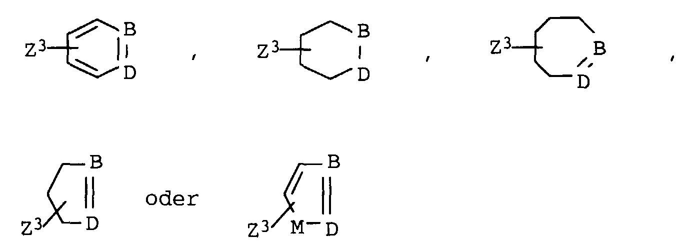 (worin Z³ eine der für Z¹ angegebenen Bedeutungen besitzt und M eine CH₂- oder NH-Gruppe ist) bedeuten,
K eine Alkylen- oder Alkenylgruppe mit jeweils bis zu 6 C-Atomen oder die Gruppe 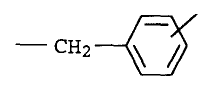 ist,
L eine Alkylen-, Alkenylen- oder Alkinylengruppe mit jeweils bis zu 6 C-Atomen oder eine der Gruppen 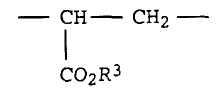 (mit R³ in der Bedeutung eines Wasserstoffatoms, einer C₁₋₄-Alkyl-, Benzyl- oder Naphthylmethylengruppe), 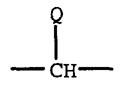 [mit Q in der Bedeutung von C₁-C₆-Alkyl, Aryl oder CH₂-R⁷, worin R⁷ Phenyl 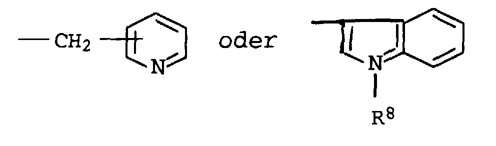 (R⁸= H, C₁₋₃-Alkyl, -CHO, -COO-C₁₋₃-Alkyl) bedeutet] oder 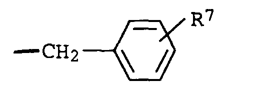
R¹ eine -CO₂R⁹-Gruppe (mit R⁹ in der Bedeutung von Wasserstoff, C₁₋₄-Alkyl oder Benzyl), -CONR⁹₂, -OR⁹, -SR⁹, -SO₃R⁹, -PO₃R⁹₂ oder einen Tetrazolrest und
R² die Gruppe 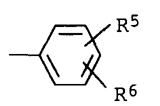 (mit R⁵ und R⁶ in der Bedeutung von Wasserstoff, C₁₋₄-Alkyl, -OR⁹ oder -SR⁹), 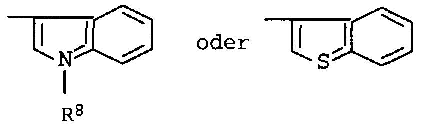
darstellen, sowie gegebenenfalls deren Salze mit physiologisch verträglichen Säuren.

3. Bicyclen-Derivate der Formel I 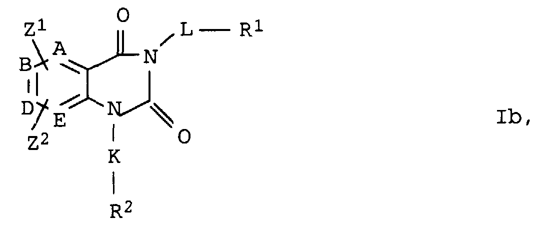 worin
2 der Reste A, B, D, E CH-Gruppen und die 2 anderen Reste CH-Gruppen oder Stickstoffatome bedeuten,
Z¹ ein Wasserstoff- oder Halogenatom, eine C₁₋₆-Alkylgruppe, eine gegebenenfalls am aromatischen Rest durch C₁₋₆-Alkyl, C₁₋₆-Alkoxy, Halogen, CF₃, NO₂ oder CN substituierte Phenyl-, Phenyl-C₁₋₄-alkylen-, Naphthyl-, Naphthyl-C₁₋₆-alkylen-Gruppe, eine C₂₋₆-Alkenyl- oder C₂₋₆-Alkinylgruppe, eine C₃₋₇-Cycloalkylgruppe oder eine der Gruppen-NHR⁴, -NR⁴₂, -OR⁴, -SO₂NHR⁴, -SO₂NR⁴₂, -COR⁴ oder -CO₂R⁴ (mit R⁴ in der Bedeutung von C₁₋₄-Alkyl, Phenyl, Phenyl-C₁₋₄-alkylen, Naphthyl oder Naphthyl-C₁₋₄-alkylen) ist,
Z² eine der für Z¹ angegebenen Bedeutungen besitzt, jedoch kein Wasserstoffatom ist, oder
Z¹ und Z² zusammen mit B und D auch einen der Reste 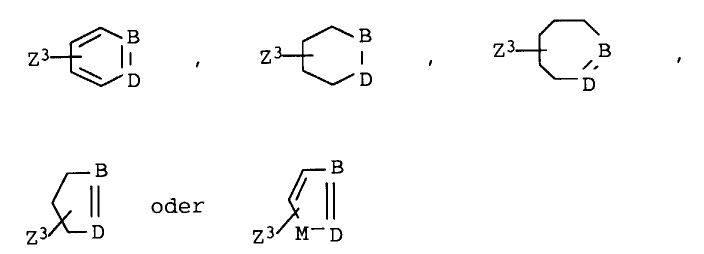 (worin Z³ eine der für Z¹ angegebenen Bedeutungen besitzt und M eine CH₂- oder NH-Gruppe ist) bedeuten,
K eine Alkylen- oder Alkenylgruppe mit jeweils bis zu 6 C-Atomen oder die Gruppe 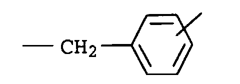 ist, 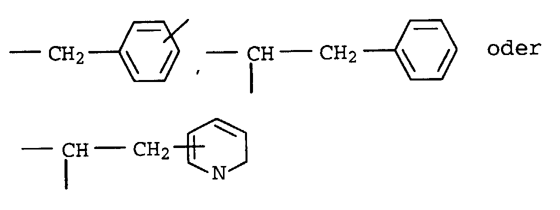
L ist,
R¹ eine -CO₂R⁹-Gruppe (mit R⁹ in der Bedeutung von Wasserstoff, C₁₋₄-Alkyl oder Benzyl), -CONR⁹₂, -OR⁹, -SR⁹, -SO₃R⁹, -PO₃R⁹₂ oder einen Tetrazolrest bedeutet und
R² die Gruppe 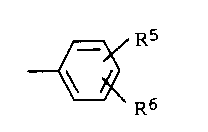 (mit R⁵ und R⁶ in der Bedeutung von Wasserstoff, C₁₋₄-Alkyl, -OR⁹ oder -SR⁹), 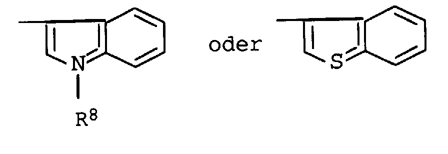 darstellt, sowie gegebenenfalls deren Salze mit physiologisch verträglichen Säuren.

## Claims

1. The use of bicycle derivatives of the formula I 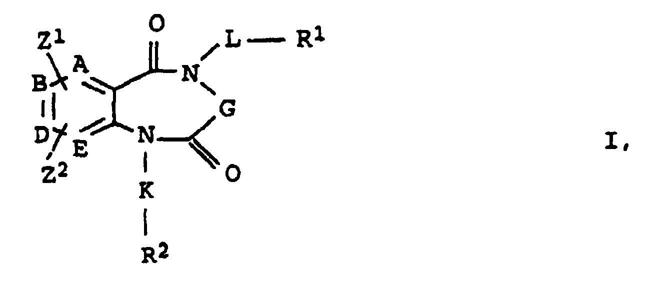 where
2 of the radicals A, B, D and E are CH groups and the 2 other radicals are CH groups or nitrogen atoms,
Z¹ is a hydrogen or halogen atom, a C₁₋₆-alkyl group, or a phenyl, phenyl-C₁₋₄-alkylene, naphthyl or naphthyl-C₁₋₆-alkylene group which is unsubstituted or substituted on the aromatic radical by C₁₋₆-alkyl, C₁₋₆-alkoxy, halogen, CF₃, NO₂ or CN, or a C₂₋₆-alkenyl or C₂₋₆-alkynyl group, a C₃₋₇-cycloalkyl group or one of the groups -NHR⁴, -NR⁴₂, -OR⁴, -SO₂NHR⁴, -SO₂NR⁴₂, -COR⁴ or -CO₂R⁴ (with R⁴ meaning C₁-₄-alkyl, phenyl, phenyl-C₁₋₄-alkylene, naphthyl or naphthyl-C₁₋₄-alkylene),
Z² has one of the meanings indicated for Z¹ but is not a hydrogen atom, or
Z¹ and Z² together with B and D are also one of the radicals 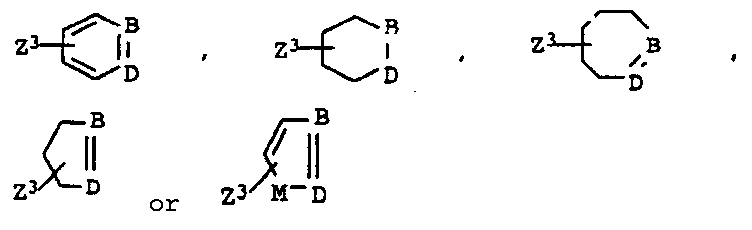 (where Z³ has one of the meanings indicated for Z¹, and M is a CH₂ or NH group),
G is a direct linkage or the group CH-K¹ (with K¹ meaning hydrogen, C₁₋₆-alkyl, or a phenyl, benzyl, naphthyl or naphthylmethylene group which is unsubstituted or substituted in the aryl moiety by C₁₋₄-alkyl, C₁₋₄-alkoxy, halogen, CF₃, NO₂ or CN),
K is an alkylene or alkenyl group with, in each case, up to 6 C atoms or the group 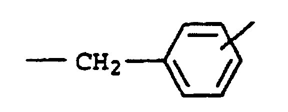
L is an alkylene, alkenylene or alkynylene group with, in each case, up to 6 C atoms or one of the groups 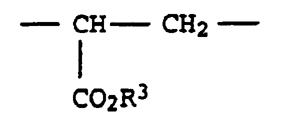 (with R³ meaning a hydrogen atom, a C₁₋₄-alkyl, benzyl or naphthylmethylene group), 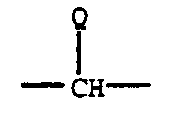 [with Q meaning C₁₋₆-alkyl, aryl or CH₂-R⁷ where R⁷ is phenyl 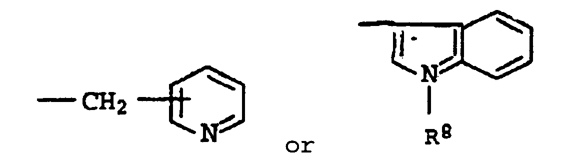 (R⁸ is H, C₁₋₃-alkyl, -CHO, -COO-C₁₋₃-alkyl)] or 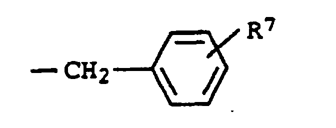
R¹ is a -CO₂R⁹ group (with R⁹ meaning hydrogen, C₁₋₄-alkyl or benzyl), -CONR⁹₂, -OR⁹, -SR⁹, -SO₃R⁹, -PO₃R⁹₂ or a tetrazole residue, and
R² is the group 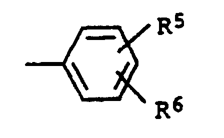 (with R⁵ and R⁶ meaning hydrogen, C₁₋₄-alkyl, -OR⁹ or -SR⁹), 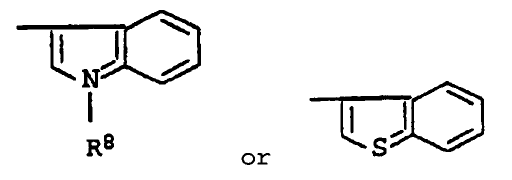
and, where appropriate, the salts thereof with physiologically tolerated acids, for the production of drugs for controlling diseases involving an elevated endothelin level.

2. A bicycle derivative of the formula I 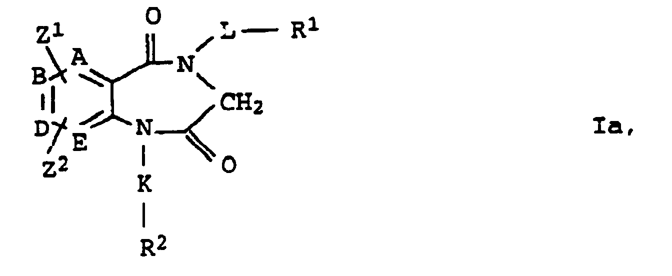 where
2 of the radicals A, B, D and E are CH groups and the 2 other radicals are CH groups or nitrogen atoms,
Z¹ is a hydrogen or halogen atom, a C₁₋₆-alkyl group, or a phenyl, phenyl-C₁₋₄-alkylene, naphthyl or naphthyl-C₁₋₆-alkylene group which is unsubstituted or substituted on the aromatic radical by C₁₋₆-alkyl, C₁₋₆-alkoxy, halogen, CF₃, NO₂ or CN, or a C₂₋₆-alkenyl or C₂₋₆-alkynyl group, a C₃₋₇-cycloalkyl group or one of the groups -NHR⁴, -NR⁴₂, -OR⁴, -SO₂NHR⁴, -SO₂NR⁴₂, -COR⁴ or -CO₂R⁴ (with R⁴ meaning C₁₋₄-alkyl, phenyl, phenyl-C₁₋₄-alkylene, naphthyl or naphthyl-C₁₋₄-alkylene),
Z² has one of the meanings indicated for Z¹ but is not a hydrogen atom, or
Z¹ and Z² together with B and D are also one of the radicals 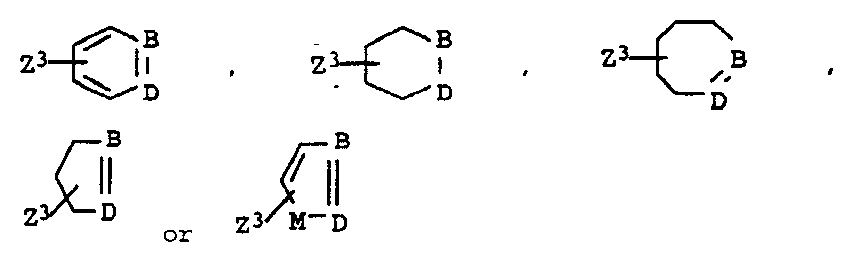 (where Z³ has one of the meanings indicated for Z¹, and M is a CH₂ or NH group),
K is an alkylene or alkenyl group with, in each case, up to 6 C atoms or the group 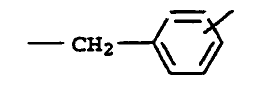
L is an alkylene, alkenylene or alkynylene group with, in each case, up to 6 C atoms or one of the groups 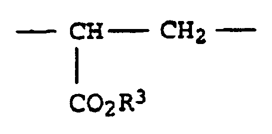 (with R³ meaning a hydrogen atom, a C₁₋₄-alkyl, benzyl or naphthylmethylene group), 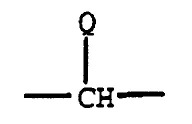 [with Q meaning C₁₋₆-alkyl, aryl or CH₂-R⁷ where R⁷ is phenyl 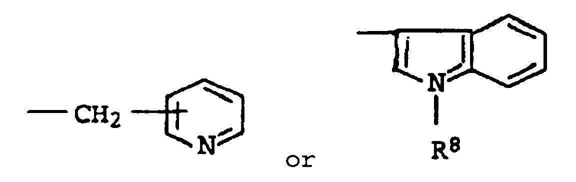 (R⁸ is H, C₁₋₃-alkyl, -CHO, -COO-C₁₋₃-alkyl)] or 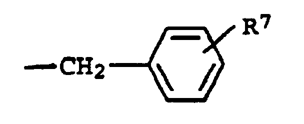
R¹ is a -CO₂R⁹ group (with R⁹ meaning hydrogen, C₁₋₄-alkyl or benzyl), -CONR⁹₂, -OR⁹, -SR⁹, -SO₃R⁹, -PO₃R⁹₂ or a tetrazole residue, and
R² is the group 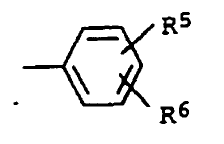 (with R⁵ and R⁶ meaning hydrogen, C₁₋₄-alkyl, -OR⁹ or -SR⁹), 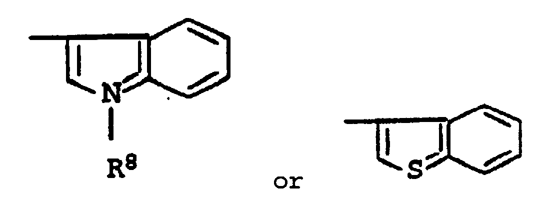
and, where appropriate, the salts thereof with physiologically tolerated acids.

3. A bicycle derivative of the formula I 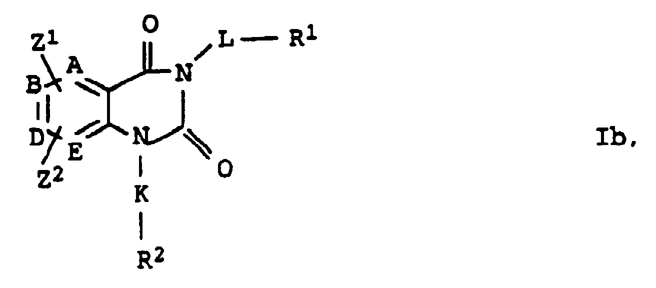 where
2 of the radicals A, B, D and E are CH groups and the 2 other radicals are CH groups or nitrogen atoms,
Z¹ is a hydrogen or halogen atom, a C₁_{*-*}₆-alkyl group, or a phenyl, phenyl-C₁₋₄-alkylene, naphthyl or naphthyl-C₁₋₆-alkylene group which is unsubstituted or substituted on the aromatic radical by C₁₋₆-alkyl, C₁₋₆-alkoxy, halogen, CF₃, NO₂ or CN, or a C₂₋₆-alkenyl or C₂₋₆-alkynyl group, a C₃₋₇-cycloalkyl group or one of the groups -NHR⁴, -NR⁴₂, -OR⁴, -SO₂NHR⁴, -SO₂NR⁴₂, -COR⁴ or -CO₂R⁴ (with R⁴ meaning C₁₋₄-alkyl, phenyl, phenyl-C₁₋₄-alkylene, naphthyl or naphthyl-C₁₋₄-alkylene),
Z² has one of the meanings indicated for Z¹ but is not a hydrogen atom, or
Z¹ and Z² together with B and D are also one of the radicals 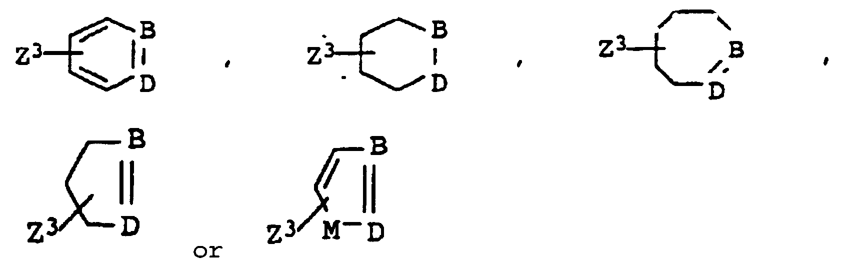 (where Z³ has one of the meanings indicated for Z¹, and M is a CH₂ or NH group),
K is an alkylene or alkenyl group with, in each case, up to 6 C atoms or the group 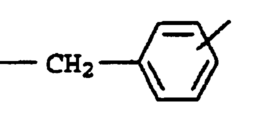
L is 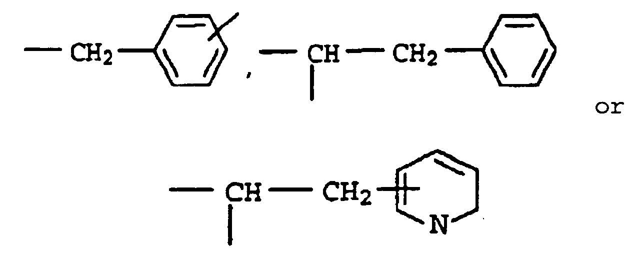
R¹ is a -CO₂R⁹ group (with R⁹ meaning hydrogen, C₁₋₄-alkyl or benzyl), -CONR⁹₂, -OR⁹, -SR⁹, -SO₃R⁹, -PO₃R⁹₂ or a tetrazole residue, and
R² is the group 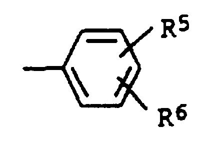 (with R⁵ and R⁶ meaning hydrogen, C₁₋₄-alkyl, -OR⁹ or -SR⁹), 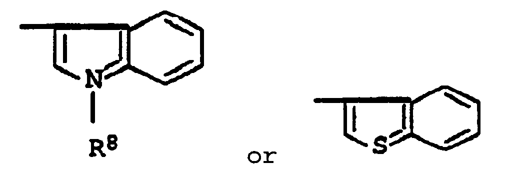 and, where appropriate, the salts thereof with physiologically tolerated acids.

## Revendications

1. Utilisation de dérivés de bicyclène de formule I 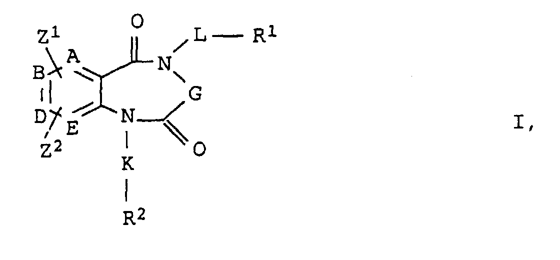 où
2 des restes A, B, D, E sont des groupes CH et les 2 autres restes représentent des groupes CH ou des atomes d'azote,
Z¹ est un atome d'hydrogène ou d'halogène, un groupe alkyle en C₁₋₆, un groupe phényle, phényl-alkylène(C₁₋₄), naphtyle ou naphtylalkylène(C₁₋₆) - éventuellement substitué sur le reste aromatique par un groupe alkyle en C₁₋₆, alcoxy en C₁₋₆, halogéno, CF₃, NO₂ ou CN-, un groupe alcényle en C₂₋₆ ou alcynyle en C₂₋₆, un groupe cycloalkyle en C₃₋₇ ou l'un des groupes -NHR⁴, -NR⁴₂, -OR⁴, -SO₂NHR⁴,-SO₂NR⁴₂, -COR⁴ ou -CO₂R⁴ (avec R⁴ qui représente un groupe alkyle en C₁₋₄, phényle, phényl-alkylène(C₁₋₄), naphtyle ou naphtylalkylène(C₁₋₄)),
Z² possède l'une des significations indiquées pour Z¹, mais n'est pas un atome d'hydrogène ou
Z¹ et Z² représentent conjointement avec B et D également l'un des restes 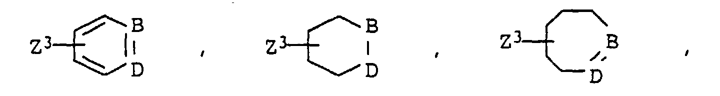 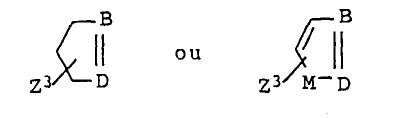 (où Z³ possède l'une des significations indiquées pour Z¹ et M est un groupe CH₂ ou NH),
G représente une liaison directe ou le groupe CH-K¹ (avec K¹ représentant l'hydrogène, un groupe alkyle en C₁₋₆ ou un groupe phényle, benzyle, naphtyle ou naphtylméthylène, éventuellement substitué dans la partie aryle par un groupe alkyle en C₁₋₄, alcoxy en C₁₋₄, halogéno, CF₃, NO₂ ou CN)
K est un groupe alkylène ou alcényle ayant dans chaque cas jusqu'à 6 atomes de carbone ou le groupe 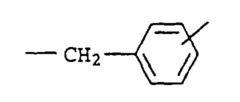
L est un groupe alkylène, alcénylène ou alcynylène ayant dans chaque cas jusqu'à 6 atomes de carbone ou l'un des groupes 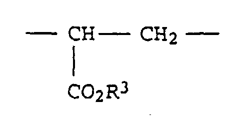
(avec R³ représentant un atome d'hydrogène ou un groupe alkyle en C₁₋₄, benzyle ou naphtylmèthylène), 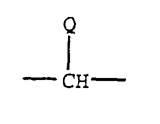 [avec Q représentant un groupe alkyle en C₁₋₆, aryle ou CH₂-R⁷, tandis que R⁷ est un groupe phényle 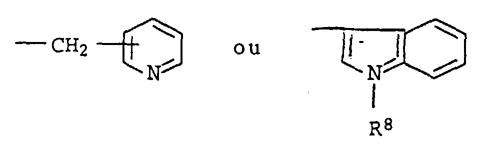 (R⁸ = H, alkyle en C₁-₃, -CHO, -COO-alkyle (C₁-₃) ou 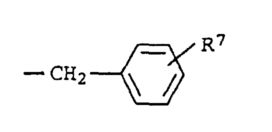
R¹ représente un groupe -CO₂R⁹ (avec R⁹ représentant l'hydrogène ou un groupe alkyle en C₁₋₄ ou benzyle), -CONR⁹₂, -OR⁹, -SR⁹, -SO₃R⁹, -PO₃R⁹₂ ou un reste tétrazole et
R² représente le groupe 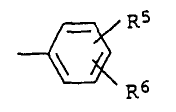 (avec R⁵ et R⁶ représentant l'hydrogène ou un groupe alkyle en C₁₋₄, -OR⁹ ou -SR⁹), 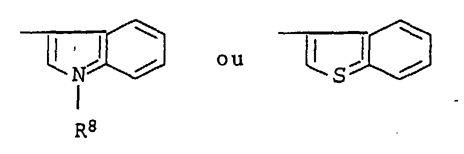 ainsi qu'éventuellement leurs sels avec des acides physiologiquement acceptables pour la préparation de médicaments pour lutter contre des maladies, qui reposent sur un niveau d'endothéline élevé.

2. Dérivés de bicyclène de formule I 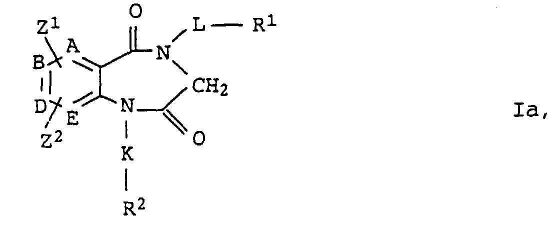 où
2 des restes A, B, D, E sont des groupes CH et les 2 autres restes représentent des groupes CH ou des atomes d'azote,
Z¹ est un atome d'hydrogène ou d'halogène, un groupe alkyle en C₁₋₆, un groupe phényle, phényl-alkylène(C₁₋₄), naphtyle ou naphtylalkylène(C₁₋₆) - éventuellement substitué sur le reste aromatique par un groupe alkyle en C₁₋₆, alcoxy en C₁₋₆, halogéno, CF₃, NO₂ ou CN-, un groupe alcényle en C₂₋₆ ou alcynyle en C₂₋₆, un groupe cycloalkyle en C₃₋₇ ou l'un des groupes -NHR⁴, -NR⁴₂, -OR⁴, -SO₂NHR⁴, -SO₂NR⁴₂, -COR⁴ ou -CO₂R⁴ (avec R⁴ qui représente un groupe alkyle en C₁₋₄, phényle, phényl-alkylène(C₁₋₄), naphtyle ou naphtylalkylène(C₁₋₄)),
Z² possède l'une des significations indiquées pour Z¹, mais n'est pas un atome d'hydrogène ou
Z¹ et Z² représentent conjointement avec B et D également l'un des restes 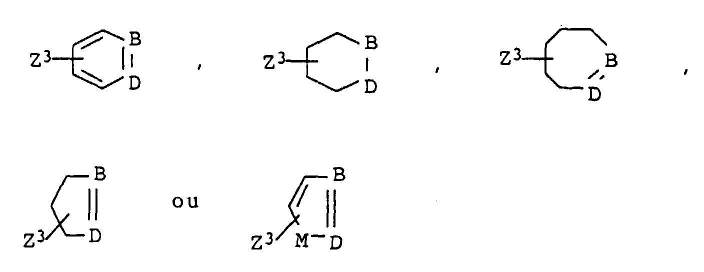 (où Z³ possède l'une des significations indiquées pour Z¹ et M est un groupe CH₂ ou NH),
K est un groupe alkylène ou alcényle ayant dans chaque cas jusqu'à 6 atomes de carbone ou le groupe 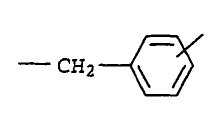
L est un groupe alkylène, alcénylène ou alcynylène ayant dans chaque cas jusqu'à 6 atomes de carbone ou l'un des groupes 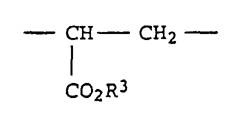
(avec R³ représentant un atome d'hydrogène ou un groupe alkyle en C₁₋₄, benzyle ou naphtylmèthylène), 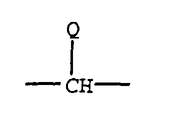 [avec Q représentant un groupe alkyle en C₁₋₆, aryle ou CH₂-R⁷, tandis que R⁷ est un groupe phényle, 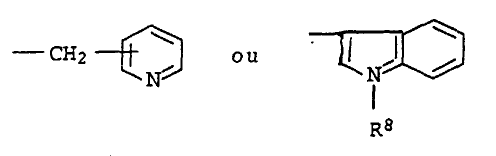 (R⁸ = H, alkyle en C₁₋₃, -CHO, -COO-alkyle(C₁₋₃)] ou 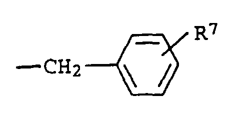
R¹ représente un groupe -CO₂R⁹ (avec R⁹ représentant l'hydrogène ou un groupe alkyle en C₁₋₄ ou benzyle), -CONR⁹₂, -OR⁹, -SR⁹, -SO₃R⁹, -PO₃R⁹₂ ou un reste tétrazole et
R2 représente le groupe 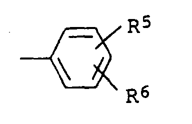 (avec R⁵ et R⁶ représentant l'hydrogène ou un groupe alkyle en C₁₋₄, -OR⁹ ou -SR⁹), 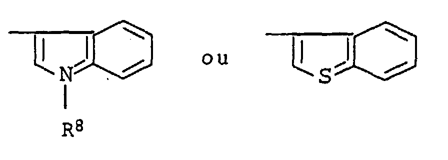 ainsi qu'éventuellement leurs sels avec des acides physiologiquement acceptables.

3. Dérivés de bicyclène de formule I 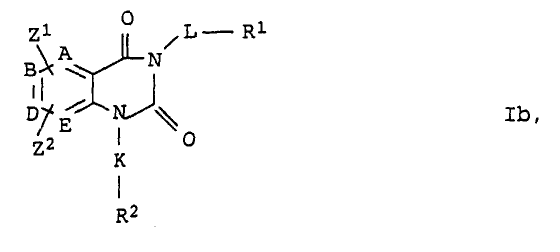 où
2 des restes A, B, D, E sont des groupes CH et les 2 autres restes représentent des groupes CH ou des atomes d'azote,
Z¹ est un atome d'hydrogène ou d'halogène, un groupe alkyle en C₁₋₆, un groupe phényle, phényl-alkylène(C₁₋₄), naphtyle ou naphtylalkylène(C₁₋₆) - éventuellement substitué sur le reste aromatique par un groupe alkyle en C₁₋₆, alcoxy en C₁₋₆, halogéno, CF₃, NO₂ ou CN-, un groupe alcényle en C₂-₆ ou alcynyle en C₂₋₆, un groupe cycloalkyle en C₃₋₇ ou l'un des groupes -NHR⁴, -NR⁴₂, -OR⁴, -SO₂NHR⁴, -SO₂NR⁴₂, -COR⁴ ou -CO₂R⁴ (avec R qui représente un groupe alkyle en C₁₋₄, phényle, phényl-alkylène(C₁₋₄), naphtyle ou naphtyl-alkylène (C₁₋₄)),
Z² possède l'une des significations indiquées pour Z¹, mais n'est pas un atome d'hydrogène ou
Z¹ et Z² représentent conjointement avec B et D également l'un des restes 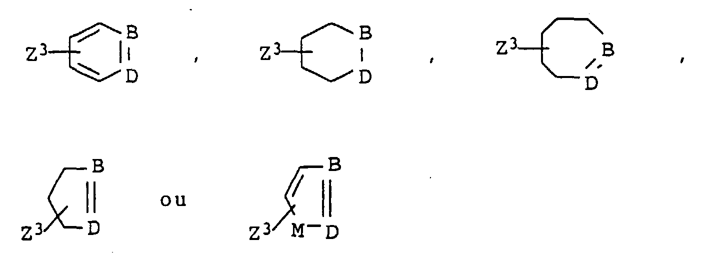 (où Z³ possède l'une des significations indiquées pour Z¹ et M est un groupe CH₂ ou NH),
K est un groupe alkylène ou alcényle ayant dans chaque cas jusqu'à 6 atomes de carbone ou le groupe 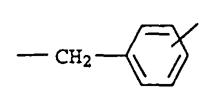
L est 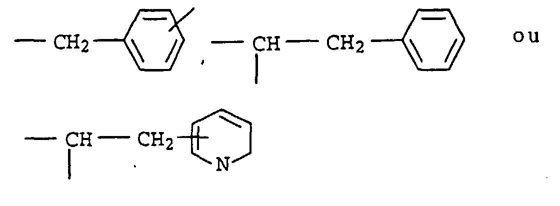
R¹ représente un groupe -CO₂R⁹ (avec R⁹ représentant l'hydrogène ou un groupe alkyle en C₁₋₄ ou benzyle), -CONR⁹₂, -OR⁹, -SR⁹, -SO₃R⁹, -PO₃R⁹₂ ou un reste tétrazole et
R² représente le groupe 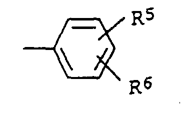 (avec R⁵ et R⁶ représentant l'hydrogène ou un groupe alkyle en C₁₋₄, -OR⁹ ou -SR⁹), 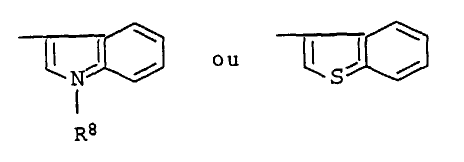
ainsi qu'éventuellement leurs sels avec des acides physiologiquement acceptables.
